# EUROPEAN PATENT APPLICATION

(11) **EP 1 674 583 A1**
(43) Date of publication of application: **28.06.2006**
(21) Application number: 04447299.1
(22) Date of filing: 23.12.2004
(51) Int. Cl.: C12Q 1/68

(54) **Method and kit for the detection of a large number of genes related to antibiotic resistance in microorganisms**

(71) Applicant: Eppendorf Array Technologies SA, B-5000 Namur (BE)
(72) Inventor: Remacle, José, 5020 Malonne (BE); Hamels, Sandrine, 1474 Ways (BE)
(74) Representative: Van Malderen, Joëlle

(57) **Abstract**

The present invention is related to a method and kit (or device) for identifying and/or quantifying in an array expressed gene nucleotide sequences related to resistance to antibiotic(s) being expressed in a (micro)organism present in a sample.

## Description

### Field of the invention

The present invention is in the field of diagnosis and is related to a method and kit comprising reagents for detection and/or quantification of related to resistance to antibiotics being potentially expressed in (micro) organisms. The detection and/or quantification of nucleotide sequences is preferably obtained after amplification.

The invention is especially suited for the identification and/or quantification of resistance from the same family and in conjunction with the identification of the (micro)organisms in the same sample.

### Background of the invention

Development of the biochips technology allows the detection of multiple nucleotide sequences simultaneously in a given assay and thus allows the identification of the corresponding organism or genes expressed by the organism. Arrays are solid supports containing on their surface a series of discrete regions bearing capture nucleotide sequences (or probes) that are able to bind (by hybridization) to a corresponding target nucleotide sequence(s) possibly present in a sample to be analyzed. If the target sequence is labeled with modified nucleotides during a reverse transcription or an amplification of said sequence, then a signal can be detected and measured at the binding location. Its intensity gives an estimation of the amount of target sequences present in the sample. Such technology allows the identification and/or quantification of genes or their expression for diagnostic or screening purpose. The invention is particularly suited to detect in a same assay resistant expressed genes together with the identification of the bacterial species present in the same sample and even together with the detection of mutations affecting genes related to the resistance.

### State of the art

The optimism of the early period of antimicrobial discovery has been tempered by the emergence of bacterial strains with resistance to these therapeutics. Today, clinically important bacteria are characterized not only by single drug resistance but also by multiple antibiotic resistances. Drug resistance presents an ever-increasing global public health threat that involves all major microbial pathogens and antimicrobial drugs.

Bacteria resistances are complex processes and are very often multiple for one bacteria or for one class of antibiotics. The main mechanisms are mutations in genes and expression of specific genes leading to resistance. Some expressed genes are specific of the antibiotic class; some are common to several antibiotic classes such as the efflux pumps.

Drug resistance is not restricted to bacteria. It is also found in fungi (Lage et al H., 2003, International journal of antimicrobial agent 22, 188-199).

To determine drug resistance, culture methods are still the protocols of reference. However, given the inherent lengthy times required by cultured methods, approaches to determined drug resistance based on molecular genetics have been developed.

The Company Affymetrix Inc. has developed a method for direct synthesis of oligonucleotides upon a solid support, at specific locations by using masks at each step of the processing. This method comprises the addition of a new nucleotide on a growing oligonucleotide in order to obtain a desired sequence at a desired location. This method is derived from the photolithographic technology and is coupled with the use of photoprotective groups, which are released before a new nucleotide is added (EP0476014, US 5,445,934, US 5,143,854 and US 5,510,270). However, only small nucleotides sequences are fixed to the surface, and the method finds applications mainly for sequencing or identifying a sequence by a pattern of positive spots corresponding to several specific oligonucleotide bound on the array. The characterization of a target nucleotide sequence is obtained by comparison of such pattern with a reference. This technique was applied to the identification of mutations within the rpoB gene that confer resistance to antibiotic drug in Mycobacterium tuberculosis (WO97/29212 and WO98/28444), wherein the capture nucleotide sequence comprises less than 30 nucleotides and from the analysis of two different sequences that may differ by a single nucleotide (the identification of SNPs or genotyping). Small capture nucleotide sequences (having a length comprised between 10 and 20 nucleotides) are preferred since the discrimination between two oligonucleotides differing in one base is higher, when their length is smaller.

The lack of sensitivity of the method is illustrated by the fact that it cannot detect directly amplicons resulting from genetic amplification (PCR). A double amplification with primer(s) bearing a T3 or T7 sequences and then a retro-transcription with a RNA polymerase. These RNA are cut into nucleotide pieces of about 40 bases before being detected on an array (example 1 of WO97/29212). However, long DNA or RNA fragments hybridize very slowly on capture sequences present on a surface. Said methods are therefore not suited for the detection of homologous sequences since the homology varies along the sequences and so part of the pieces could hybridise on the same capture sequences. Therefore, a software for the interpretation of the results should be incorporated in the method for allowing interpretation of the obtained data.

However, for gene expression array, which is based on the cDNA copy of mRNA the same problem is encountered when using small capture probe arrays: the rate of hybridization is low. Therefore, the fragments are cut into smaller species and the method requires the use of several capture nucleotide sequences in order to obtain a pattern of signals, which attest the presence of a given gene (WO97/10364 and WO97/27317). Said cutting also decreases the number of incorporated labeled nucleotides, and thus reduces the obtained signal. The use of long capture nucleotide sequences would give a much better sensitivity for the detection. In the many gene expression applications, the use of long capture probes is not a problem, when cDNA to be detected originates from different genes having non homologous, sequences, since there is no cross-reactions between them. Long capture nucleotide sequences give the required sensitivity, however, they will be able to hybridize to other homologous sequences.

The patent application WO9961660 provides another method for detecting macrolide antibiotic resistances in microorganisms by *in situ* hybridization of probes targeting mutations in rRNA 16S and 23S. The samples are analyzed by microscopy.

In eukaryotes, mRNA measurements utilizing microarray has been facilited by making cDNA copies of mRNA using polyA tail-specific primer. The lack of a polyA tail and the extremely short bacterial mRNA half-life represent hurdles for the application of DNA microarray technology to prokaryotic research. Adaptation of RNA isolation and labelling protocol from eukaryotes to prokaryotes is not straightforward since eukaryotic mRNA manipulation often exploits 3' polyadenylation of this molecular species which is not present in prokaryotic organisms.

Identification of gene expression in *E*. *coli* has been proposed in the patent application W00129261A2 using a high-density microarray prepared with a collection of ORFs of *E*. *coli* genome. The global effect on genes under different environmental conditions is determined by a gene expression profiling and a comparison to a control sequence. Labeled cDNA were obtained by using random hexamer primers regardless the presence of polyadenylation and were hybridized without amplification to the high density microarray. The method is restricted to the bacterial species for which the sequences of the microarray have been constructed and is labor intensive since the microarray contains at least 75% of all ORF in the bacterial species. As the genes associated with resistance to antibiotic drug in bacteria are usually expressed at a very low level, their evidence requires amplification of the mRNAs.

There is a growing need to detect not only the fact that one bacteria is resistant but also to know the mechanism of resistance and to understand the relation between the resistance acquired in one bacteria to the resistance to another of the same species and to one of other species. The transmission of resistance from one bacteria to another one is a main concern in epidemiology studies and in public health since it allows to control the spread of resistance among some bacteria or in a given physical or geographical place. One of the most sensitive place is the hospital where resistance bacteria are selected due to the continuous presence of antibiotics.

### Aims of the invention

The present invention aims to provide a new method and kit or device that do not present the drawbacks of the state of the art and that improve microarrays and biochips technology for the easy identification (detection and/or quantification) in a sample of a large number of portions (nucleotide sequences) of (micro)organisms having homologous nucleotide sequences, in particular nucleotide sequences involved in resistance of the (micro)organism to antibiotics when expressed in these (micro)organisms.

A further aim of the invention is to provide such method and device which are based upon a simplified technology requiring only the use of one or more consensus primer(s) in copy and/or amplification steps and which allow the identification, the quantification and/or the recording of a single spot signal upon said microarray, said single spot signal resulting only from the specific binding of the target sequence to its corresponding capture sequence.

Another aim of the invention is to provide a method for the simultaneous detection and/or quantification of antibiotic resistance genes expressed in the (micro)organism as well as genomic DNA of (micro) organism, especially genes allowing further the identification and/or quantification of the (micro)organism.

### Definitions

The terms "nucleic acid, oligonucleotide, array, probe, target nucleic acid, bind substantially, hybridizing specifically to, background, quantifying" are the ones described in the international patent application WO97/27317 incorporated herein by reference.

The terms "nucleotide triphosphate, nucleotide, primer sequence, Homologous sequences, consensus sequence" are those described in the US patent application US2002/106646A1 incorporated herein by reference.

Methods of alignment of sequences are based on local homology algorithms which have been computerised and are available as for example (but not limited to) Clustal® , (Intelligenetics, Mountain Views, California), or GAP® , BESTFIT® , FASTA® and TFASTA® (Wisconsin Genetics Software Package, Genetics Computer Group Madison, Wisconsin, USA) or Boxshade® .

The consensus sequence represents a sort of «average» sequence, which is as close as possible from all the compared sequences. For high homologous sequences (which means sequences presenting an important identity of sequence between them), if the consensus sequence is long enough and the reaction conditions are not too stringent, it can bind to all the homologous sequences. This is especially useful for the amplification of homologous sequences with the same primers called, consensus primers. Experimentally, the consensus sequence calculated from the alignment programs above can be slightly adapted in order to obtain such property. Variations do not exceed 50% from the calculated sequence.

As used herein, the term "capture molecule" refers to a molecule, or complex or combination thereof, that is capable of specifically binding to one target molecule, or to a family of target molecules, or to one or more member(s) of a plurality of target molecules, or portion(s) thereof. The capture molecules are preferably nucleic acids or equivalent molecules such as PNA which are either synthesized chemically in situ on the surface of the support or laid down thereon. Nucleic acid binding is achieved via base pairing between two polynucleotides, one being the immobilized capture molecule and the other one the target to be detected.

The term "microarray" means an insoluble solid support on which multiple capture sequences are immobilized in order to be able to bind to the given specific target sequence. A sequence that can specifically bind another sequence means a specific hybridization binding by complementary nucleotide sequences. This array is preferentially composed of capture sequences present at specifically localized areas (specific locations) on the solid support surface or within the support or on a substrate covering the support. A specifically localized area is the area of the surface, which contains bound capture nucleotide sequences, said capture nucleotide sequence being specific for a determined target sequence. The area wherein the given capture sequence (bound to a target sequence) is fixed and seen (detected) by a detector is called a "spot" producing a specific signal detected at this localized area. In one particular embodiment of this invention, arrays of capture sequences are also provided on different supports as long as these different supports contain specific capture sequences and may be distinguished from each other in order to be able to detect and/or quantify these specific corresponding target molecules. This can be achieved by using a mixture of beads having particular features and being able to be recognized from each other in order to quantify the bound sequences. One bead, or a population of beads is then considered as a "spot" having at least one bound capture sequence specific of a target sequence.

The term "signal" resulting from a specific binding at a specific location means a detection and possibly a quantification of a single hybridization event between complementary nucleotide sequences at the specific localized area (location) of a fixed capture sequence of the solid support surface (or inside the solid support). A complementary hybridization can be detected and possibly quantified by the (fluorescent, colorimetric, etc.) label introduced in the sequence of the target sequence or at the extremity (preferably during the copy or amplification step)of the target sequence.

The term "expressed genes" are those regions of the genomic DNA which are transcribed into mRNA and optionally then translated into (poly)peptides or proteins. According to the present invention the determination of expressed genes is performed via detection of these mRNA sequences.

The term "(micro)organism" relates to microbial entities as such, e.g. bacteria or fungi, and comprises parts thereof such as the expressed genes in the form of RNA. Since RNA are rapidly degraded, the present invention mostly detect the genes of living organisms.

### Summary of the invention

The invention relates to an identification and/or quantification method of a part of a biological (micro)organism possibly present in a biological sample by the detection of the expressed gene nucleotide sequences related to resistance to antibiotic(s) and being expressed in the (micro)organism, wherein said nucleotide sequence is homologous to at least 4 other nucleotide sequences from other biological (micro)organisms and comprising the steps of :
- extracting the expressed gene nucleotide sequences from the (micro)organism;
- adding enzymatically a preferably homopolymeric nucleotide tail to at least one extremity of the said sequence (an homopolymeric tail contains a stretch of at least 4 and preferably 10 nucleotides of the same base in a continuous sequence);
- copying and/or amplifying at least part of the said sequence into target homologous nucleotide sequences to be detected using unique primer(s) (preferably an unique pair of primer(s), which are capable of amplifying and/or copying at least 4 homologous target nucleotide sequences. For classical amplification steps, such as the one proposed by PCR, a pair of primer (s) is used while for other copying steps such as IVT above described, a single primer may be used;
- possibly labelling said target nucleotide sequences (preferably the labelling of the target nucleotide sequences is done during the copying and/or amplifying step);
- putting into contact the (labelled) target nucleotide sequence with single stranded capture nucleotide sequences bound by a single pre-determinate link to the insoluble solid support, preferably a non-porous solid support;
- discriminating the binding of the target nucleotide sequences specific (of a part of the (micro)organisms by detecting and/or recording a signal resulting from an hybridisation by complementary base pairing between the target nucleotide sequence and its corresponding capture nucleotide sequence,
- wherein the said capture nucleotide sequences are bound to the insoluble solid support at specific locations according to an array, having a density of at least 4 different bound single stranded capture nucleotide sequences/cm2 of the solid support surface and wherein the binding between the target nucleotide sequences and its corresponding capture nucleotide sequence forms (will result in) said signal at the expected location, the detection of a single spot signal allowing to indicate directly (results not requiring a complicate and expensive further analysis by a software) present or absence of an expressed nucleotide sequence and predict a possible antibiotic(s) resistance to the (micro)organisms, expressing said nucleotide sequences.

In the method according to the invention the target sequence present between them an homology of sequence comprised between 2% and 80% and wherein the single stranded capture nucleotide sequence has at least a length of 50 nucleotides, preferably a length of 200 nucleotides, but preferably lower than 500 nucleotides, more preferably lower than 300 nucleotides; said single stranded capture nucleotide sequence being able to specifically bind to a target nucleotide sequence to be detected and/or quantified without any binding to said at least 4 other different homologous nucleotide sequences having an homology comprised between 2% and 80% with the said target sequence to be detected and/or quantified.

According to another embodiment of the method of the invention, the said target homologous nucleotide sequence present between them a homology of sequence (sequence identity) higher than 80%, but lower than 99%.

In said embodiment, it is necessary that the single stranded capture nucleotide sequence comprises a specific portion of nucleotide sequences of about 10 to about 50 nucleotides, more preferably between 15 and 30 nucleotides, said specific portion being able to specifically bind (by hybridisation) to the target nucleotide sequence to be detected and/or quantified without having any binding to said at least 4 other different nucleotide sequences having a homology higher than 80% with the said target nucleotide sequence to be detected and/or quantified. Furthermore, the said specific portion of the capture nucleotide sequence is fixed at a certain distance from the solid support surface, preferably via a spacer and said spacer is preferably a nucleotide sequence of at least 20 nucleotides, more preferably at least 40 nucleotides, more preferably at least 90 nucleotides.

According to another embodiment of the method according to the invention, it is possible to propose simultaneously a different detection of sequences presenting homology between 2% and 99%. Therefore, the array comprises two groups of single stranded capture nucleotide sequences :
- a first group composed of capture nucleotide sequences having a nucleotide sequence comprised between 10 and about 50 nucleotides, preferably between 15 and 30 nucleotides which is able to able to specifically bind to the target nucleotide sequence to be detected without binding to said at least 4 other different homologous nucleotide sequences having an homology higher than 80% with the said target nucleotide sequence, wherein the capture nucleotide sequences able to hybridize with the corresponding target sequence is separated from the surface of the solid support by a spacer as above described, preferably a spacer being a nucleotide sequence having at least 20 nucleotides, preferably at least 40 nucleotides, more preferably at least 90 nucleotides. In the method according to the invention, the array comprises a second group composed of capture nucleotide sequences comprising a nucleotide sequence of at least 50 nucleotides, more preferably at least 200 nucleotides, but lower than 500 nucleotides which is able to specifically bind to the target nucleotide sequence to be detected and/or quantified without binding to said at least 4 different nucleotide sequences having an homology comprised between 2% and 80% with the said target nucleotide sequence.

In the method according to the invention, several expressed genes nucleotide sequences related to resistance to antibiotic(s) being expressed in a (micro)organism and being present in the same sample, are simultaneously identified and/or quantified by the above-mentioned steps.

In the method according to the invention, at least 2 expressed gene nucleotide sequences related to resistance to antibiotic(s) are simultaneously identified and/or quantified, said sequence belongs to two families comprising firstly the sequence specific for one antibiotic, and secondly, the sequence common for several antibiotic(s) with at least one sequence being identified and/or quantified in each family, preferably the method applied for the detection of at least 10 expressed gene nucleotide sequences with at least 6 sequences being specific for one antibiotic and at least 4 sequences common for several antibiotic(s) being identified and/or quantified by the method of the invention. The specific sequences for one antibiotic being present in the tables I, II, III, V and VI and the sequences common for several antibiotics being present in the tables VII and VIII. These genes being selected from the group consisting of methylase enzymes, acetyltransferases, phosphostransferases, adenyl-transferases, ligase enzymes, tet proteins, B-lactamase enzymes,efflux transporters and inactivating enzymes, preferably esterase, hydrolase, transferase and phosphorylase.

In the method according to the invention, the step of copying and/or amplifying at least part of the expressed gene nucleotide sequences is obtained with the addition of an unique primer being the complement of homopolymeric nucleotide tail added to one extremity of the expressed gene nucleotide sequence; preferably, at least the 5' sequence extremity or the 5' and 3' sequences extremities. Said unique primer has a length of at least 10 nucleotides or may comprises a T7 RNA promoter nucleotide sequence or its complementary nucleotide strand. The amplifying step could be an in vitro transcription (IVT) or a PCR.

In the method and diagnostic and/or quantification kit or device according to the invention, the insoluble solid support is selecting from the group consisting of glasses, electronic devices, silicon supports, plastic supports, compact discs, gel layers, metallic supports or a mixture thereof.

Furthermore, the method according to the invention may also comprise,the steps of an identification of a single mutation in a target nucleotide sequence related to resistance to antibiotic(s), said single nucleotide mutation could be detected by using two types of capture molecules able to bind said target nucleotide sequence, said capture nucleotide sequence differing by a single base, the detection of said mutation being obtained by a preferred binding of the target nucleotide sequence upon the capture molecules comprising the single base mutation.

Furthermore, the expressed gene nucleotide sequences related to resistance to antibiotic(s) are firstly separated from eukaryotic genes before submitted to the copy and/or amplification step.

Another aspect of the present invention is related to a diagnostic and/or quantification kit or device which comprises the means and media for performing the method according to the invention, preferably an insoluble solid support upon which two groups of single stranded capture nucleotide sequences are bound according to an array wherein said first group being composed of capture nucleotide sequences comprising a specific portion of nucleotide sequences having a length of about 10 to 50 nucleotides, preferably between 15 and 30 nucleotides which is able to specifically bind a target nucleotide sequence related to resistance to antibiotic(s) to be identified and/or quantified without binding at least 4 other homologous and different nucleotide sequences having an homology higher than 80% with the said target nucleotide sequence, said specific portion of the capture nucleotide sequence being fixed to the solid support surface at a certain distance from said solid support surface, preferably via a spacer of at least 20 nucleotides, preferably at least 40 nucleotides, more preferably at least 90 nucleotides; and wherein said second group being composed of capture nucleotide sequences having at least 50 nucleotides, preferably at least 200 nucleotides which is able to specifically bind to a target nucleotide sequence related to resistance to antibiotic(s) to be identified and/or quantified without binding to at least 4 other homologous and different nucleotide sequences having an homology comprised between 2% and 80% with the said target nucleotide sequence.

In the kit and method according to the invention, the homologous and different nucleotide sequences could be present simultaneously in the sample biological sample and the said capture nucleotide sequences are covalently bound to the solid support in view to form an array which comprises at least 4 different bound capture nucleotide sequences / cm² surface fixed at specific locations on the solid support.

According to another embodiment of the present invention, the diagnostic and/or quantification kit or device according to the invention may comprise an insoluble solid support upon which are covalently bound capture nucleotide sequences according to an array which comprises at least 4 different bound capture nucleotide sequences / cm² surface fixed at specific locations on the solid support of a part of a (micro)organism to be detected and/or quantified, said part being an expressed gene nucleotide sequence related to resistance to antibiotic(s) to be identified and/or quantified.

In the method according to the invention, the nucleotides have a length of at least 50 nucleotides, preferably at least 200 nucleotides, but less than 500 nucleotides, preferably less than 300 nucleotides.

Advantageously, expressed genes related to (micro)organism species and/or genus and their resistance to antibiotics are simultaneously identified and quantified on the same support.

The invention is also related to the identification of mutation present in the resistant related genes. Identification of a single nucleotide polymorphism is obtained trough the use of at least 2 capture molecule differing by one single base for questioning the mutation position to be identified.

The (micro) organisms could be present in any biological sample including genetic material obtained (virus, fungi, bacteria, plant or animal cell, including the human). The biological sample is also any culture medium wherein microorganisms, xenobiotics or pollutants are present, as well as such extract obtained from a plant or an animal (including a human) organ, tissue, cell or biological fluid (blood, serum, urine, etc).

The kit preferably also includes the array further comprising capture nucleotide sequences for the identification of mutations in target nucleotide sequences related to resistance to antibiotic(s). The kit also preferably contains the reagents and solution necessary for making the hybridization, also the controls and standards and preferably the labeled molecules.

Extraction means any kind of isolation of genetic material (mRNA, genomic DNA) from the sample by physical or chemical process. Detection in cell culture, is preferred performed on mRNA purified from the (micro)organism directly after extraction of total RNA.
Assays on (micro)organisms extracted from a biological sample or from clinical sample preferably required the elimination of contaminating mammalian RNA before the purification of the (micro)organism mRNA. This step is preferably by commercially available kits so as the Ambion's MICROBEnrich Kit (Ambion, Cambridgeshire, UK) in which magnetic beads derivatized with oligonucleotides capture and remove mammalian RNA (18S rRNA, 28S rRNA and polyadenylated mRNA) from host bacteria RNA mixture.

A nucleotide tail means a polynucleotide made of nucleotides which are enzymatically synthesized or chemically added to at least one extremity of the expressed gene nucleotide sequence. The tail is preferably a homopolymeric nucleotide when using enzymatic synthesis. If the tail is chemically added, it can also be polynucleotide of known sequence. The homopolymer contains a stretch of at least 4 preferably 10 nucleotides of the same base in a continuous sequence.

Unique primer means a primer unique for the amplification or copy of the gene nucleotide sequences of interest. This unique primer is compatible with other primers when necessary for gene copy and/or amplification beside the one described here. Unique primer(s) also includes a family of primer(s) having in common a given sequence for hybridization to a common nucleotide sequence present on the sequences to be copied or amplified. Typical unique primer is poly-dT having a continous sequence of between 7 and 50 dT. Typical unique primer family is poly-dT as above being terminated at their 3' end by one or a small number of other nucleotides. Other unique primer family is also a primer having one or 2 or even 5 degenerated bases which account for the small variability of the target sequences compared to the consensus primer sequence.

Labelling of the targets is obtained during the copy or amplification of the nucleotide sequences preferably by the incorporation of labelled dNTP during the synthesis. Alternatively, labelling is performed after the copy or amplification preferably by the Kreatech labeling kit (ULS labeling, kit, Kreatech, Amsterdam, The Netherland) . Advantageously, the longer is the copied or amplified sequence, the more markers are present on the hybridized target making the assay sensitive.

The single stranded capture nucleotide sequences are advantageously covalently bound (or fixed) upon the insoluble solid support, preferably by one of their extremities.

A full target nucleotide means at least 90% of the nucleotide sequence which has been copied and/or amplified. This means that the target nucleotides are not fragmented before hybridization.
Preferably, specific hybridisation is obtained under stringent conditions (under conditions well-known to the person skilled in the art, for instance, the one described by Sambrook et al) which provides sufficient binding of the target sequences on their specific capture probes to the detected with no or very low, preferably lower than 5% and even lower than 1%, cross-hybridization on non-related target capture probes.

### Brief description of the drawings

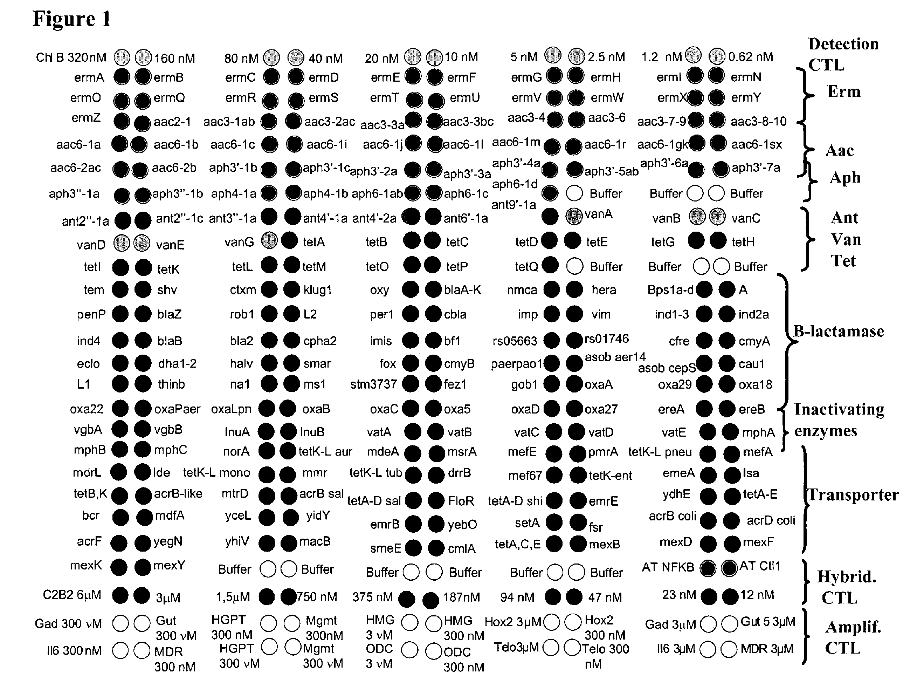

The figure 1 is a schematic presentation of the design of the microarray for identification of antibiotic resistance gene expressed in bacteria. Part of the mRNA of *erm* (subclasses A to I, N, O, Q to Z), aac (subclasses 2, 3, 6), aph (subclasses 3', 3", 4, 6), *ant* (subclasses 2", 3", 4", 6", 9"), *van* (subclasses A to E, G), tet (subclasses A to E, G to I, K to M, O to Q) □-*lactamase enzymes* (subclasses A.1 to A.5, B.1 to B.5, C.1 to C.4, E.1 to E.2, D.1 to D.5), *inactivating enzymes* {ere (A, B), *vgb* (A, B), *lnu* (A, B), vat (A to E), *mph* (A to C)}, *efflux transporter* (ABC, MATE, MSF, RND, SMR) are copied, amplified and detected by the method of the invention. Various controls are present on the array including amplification, hybridization and detection controls (CTL).

### Detailed description of the invention

In a preferred embodiment, the method of the invention related to the identification and/or quantification of a gene related to resistance to antibiotics being expressed in a (micro)organism among at least 3 different other resistant related genes having an homology of sequence comprised between 2 and 80%.

In the main embodiment, target and/or capture molecules are biological molecules nucleic acids attached preferably by covalent link on some parts of the surface of The support. A specifically localized area is the area of the surface which contains bound capture molecules specific for one determined target molecule. In another embodiment, the capture molecules are adsorbed on the support as long as they are not significantly released in solution during the detection.

Deposition of the capture probe is preferentially done with physical means such as pin or "pin and ring" touching the surface, or by release of a micro-droplet of solution by methods such as piezo or nanodispenser or similar methods for as long as specific capture molecules are present in specific (specific location) localized area.

Localized area for the detection of the targets are preferably comprised between 1 micron² and 1mm² Capture nucleotide sequences used for the binding each different target nucleotide sequence have length comprised between 50 and 1000 nucleotides, preferably between 200 and 400 nucleotides. Advantageously, long capture nucleotide sequences of at least 50 nucleotides, preferably of at least 200 nucleotides hybridize with high yield on their specific capture nucleotide sequence.

Detection of target nucleotide having homology higher than 80% are detected together on long capture nucleotide sequences used advantageously as "consensus" sequences for detection of several target sequences belonging to the same subclass or family of gene related to resistance to antibiotics. Indeed, most of the time it is not necessary to discriminate subclasses or families of genes related to resistance to antibiotics to identify the resistance of the (micro)organism to antibiotic(s). An example of such advantage is illustrated in Table II. Probe aac(3)2a-c is consensus for target sequences aac(3)2a, 2b and 2c. If, the array comprises only the necessary number of capture nucleotide sequences, the conception of the array is highly simplified as well as the data analysis after the experiment has been performed.

Detection and discrimination between target nucleotide sequences having homology higher than 80% is performed on microarray having capture nucleotides being nucleotide sequences of about 10 to about 50 nucleotides which are able to specifically bind to target nucleotide sequences without binding to at least 3 different nucleotide sequences having an homology higher than 80% and are fixed to a support via a spacer of at least 20 nucleotides.

In a preferred embodiment, the capture nucleotide sequences comprises a nucleotide sequence of about 15 to about 30 bases which is able to specifically bind to said target nucleotide. These bases are preferably assigned as a continuous sequence located at or near the extremity of the capture nucleotide sequence. This sequence is considered as the specific sequence for the detection. In a preferred form of the invention, the sequence located between the specific capture nucleotide sequence and the support is a spacer which has a nucleotide sequence non related to the target nucleotide sequences preferably having the sequence according to the patent W00177372.

In a preferred embodiment of the invention, a spacer of at least 40 nucleotides and ever better at least 90 nucleotides is linked the specific nucleotide sequence and is fixed on the support by one of its free extremity preferably the 5'end.

In the preferred embodiment, the polynucleotides being used as capture molecules are present on the microarray localized area at a density superior to 3, fmoles, and preferably 100 fmoles per cm² surface of the solid support.

In another embodiment of the invention, the capture nucleotide sequences are chemically synthesised single stranded oligonucleotides sequences shorter than 100 nucleotides (easily performed on programmed automatic synthesiser). Such sequences are either synthesized in situ on the surface of the support or are deposit upon the support, at high concentrations and they bear a functionalised group for covalent attachment.

Capture polynucleotide sequences longer than 100 nucleotides are preferably synthesised by PCR amplification (of a sequence incorporated into a plasmid containing the specific part of the capture nucleotide sequence and possibly the non specific part (spacer)). Capture polynucleotide sequences between 100 and 200 nucleotides long are also possibly single stranded DNA chemically synthesized.

Capture nucleotide sequences for the identification and/or quantification of a gene related to resistance to antibiotics being expressed in a (micro)organism are preferably located closed to the 3' end of the mRNAs. The nucleotide tail is preferably made of homopolymeric bases and is preferably added to the 3' of the mRNAs. Distance to the added nucleotide tail is optimized in order to ensure that reverse transcription efficiency is sufficient to cover the region where the capture nucleotide sequences are placed.

In a preferred embodiment, the method of the invention related to the identification and/or quantification of a gene related to resistance to antibiotic(s) being expressed in a (micro)organism among at least 3 different other resistant related genes having an homology of sequence comprised between 2 and 99%, wherein the array comprises two groups of single-stranded capture nucleotide sequences: a first group composed of capture nucleotide sequences comprising a nucleotide sequence of about 10 to about 50 nucleotides which is able to specifically bind to a target nucleotide sequence without binding to said at least 3 different nucleotide sequences having an homology higher than 80% and wherein the first capture nucleotide sequences are fixed to a support via a spacer of at least 20 nucleotides and a second group composed of capture nucleotide sequences comprising a nucleotide sequence of at least 50 nucleotides which is able to specifically bind to a target nucleotide sequence without binding to said at least 3 different nucleotide sequences having an homology comprised between 2 and 80%.

Advantageously, this embodiment of the invention allows the identification and/or quantification on the same array of nucleotide sequences having homologies higher than 80% and lower than 80% as compared to other nucleotide sequences present in the same sample. This is particularly useful for the combination on the array of "consensus" capture nucleotide sequences which will detect several target sequences belonging to the same subclass or family of gene related to resistance to antibiotics with capture nucleotide sequences specific of subclasses or families of genes.

Another preferred embodiment of the invention relates to a method for the identification of a (micro)organism species and/or genus and their resistance to antibiotic(s) in a sample being possibly contaminated with one or more of at least 3 different other (micro)organism species and/or genus and possibly containing 3 other resistant genes.

The identification is preferably obtained after copy and/or amplification of expressed genes nucleotide sequences related to (micro)organism species and/or genus and their resistance to antibiotic(s) into target nucleotide sequences using a unique pair of primer(s) for the 4 (micro)organisms species and/or genus genes and the 4 resistant genes possibly present.

In an alternative embodiment, the (micro)organism species and/or genus are identified by amplification of one or more parts of their genomic DNA into target nucleotide sequences using a unique pair of primer(s) for the 4 (micro)organisms species and/or genus genes possibly present. The resistance to antibiotic(s) is identified by copy and/or amplification of expressed gene nucleotide sequences using a unique pair of primer(s) into target nucleotide sequences wherein one primer at least is unique primer for the 4 resistant genes. Target nucleotide sequences are identified on the same array comprising two groups of single-stranded capture nucleotide sequences: a first group composed of capture nucleotide sequences being fixed to a support via a spacer of at least 20 nucleotides and comprising a nucleotide, sequence of about 10 to about 50 nucleotides which is able to specifically bind to said (micro)organism target nucleotide sequence without binding to said at least 3 other (micro)organisms nucleotide sequences and a second group composed of capture nucleotide sequences comprising a nucleotide sequence of at least 50 nucleotides which is able to specifically bind to said resistant target nucleotide sequence without binding to said at least 3 other resistant nucleotide sequences. The single-stranded capture nucleotide sequences are covalently bound to an insoluble support and the array comprises at least 4 different bound single-stranded capture nucleotide sequences /cm2 fixed at specific locations of the support.

The capture nucleotide sequences of the first group are preferably used for the capture of target nucleotide sequence having homologies higher than 80% with other related target nucleotide sequences while capture nucleotide sequences of the second group are better used for the capture of target nucleotide sequence having homologies lower than 80% with other related target nucleotide sequences.

In a preferred embodiment, the step of copying at least part of expressed gene nucleotide sequences is performed using a unique primer being the complement of nucleotide tail added to one extremity of the expressed gene nucleotide sequences. The unique primer is at least 10 nucleotides and preferably comprised between 15 and 50 nucleotide long.

In another preferred embodiment, the step of amplifying at least part of expressed gene nucleotide sequences is performed using in vitro transcription (IVT). The unique primer that is used for synthesis of the first cDNA strand comprises a promoter for a T7 RNA polymerase or its complement. After synthesis of the second cDNA strand, addition of T7 RNA polymerase allows the production of multiple copies antisense RNA (aRNA). The amplification is linear so that quantification of the mRNA remains possible after amplification. Labelling of the aRNA is possible during its synthesis or after it. Labelled aRNA can be directly contacted with the array. Such embodiment requires working in strict RNase free conditions and in the presence of denaturing agents to unfold secondary structures of the aRNA.

In a preferred embodiment, aRNA are further copied into complementary single stranded or double stranded cDNA using a second primer for the targets to be detected. Labelling of the cDNA is possible during its synthesis or after it. Labelled cDNA can be contacted with the array without requirement of RNase free conditions. The capture probes of the array are the complement strand at least in part of the labelled cDNA. If the labelled cDNA is single stranded, the capture probes have to be anti-sense oriented as compared to the mRNA strand. If the labelled cDNA is double stranded, the use of sense or anti-sense oriented capture probes is possible.

In an alternative embodiment, the step of amplifying at least part of expressed gene nucleotide sequences is performed using polymerase chain reaction (PCR). One primer of the unique pair is used for synthesis of cDNA. It is preferably the complement of the nucleotide tail added to one of the extremity of the mRNA, preferably the 3'end. It contains poly(dT) streches if a poly(A) has been added to the mRNA. In a preferred embodiment, a homopolymeric nucleotide tail is added to the 3' end of the cDNA, preferably a poly(dC). Such tail is preferably synthesized by the terminal transferase which is able to add a nucleotide tail to single stranded DNA fragments. The length of the tail is preferably comprised between 10 and 40 nucleotides.

In a preferred embodiment, the unique pair of primers for PCR comprised a stretch of poly(dT) and a stretch of poly (dG) of at least 8 homonucleotides. Labelling of the amplicons is possible during its synthesis or after it. If both strands of amplicons are labelled, the use of sense or anti-sense oriented capture probes is possible.

The identification of resistance genes for specific antibiotic does not required acute quantification of the target mRNAs and usually semi-quantification or the simple detection of the expressed gene is sufficient. However, for some genes especially the efflux transporter resistant genes quantification is required. When quantification is required, the IVT is preferred as amplification step due to the linearity of the amplification. In a preferred embodiment, internal standards of known concentration are used for quantification. They are preferably mRNA having a poly(A) at their 3'. Internal standards are added to the IVT product (aRNA) and are simultaneously copied into cDNA.

In a preferred embodiment, the insoluble support of the method and kit of the invention is selected from the group consisting of glasses, electronic devices, silicon supports, plastic supports, compact discs, gel layers, metallic supports or a mixture thereof.

In another preferred embodiment, the support bearing the capture molecules has a 3 dimensional porous structure. Conventional glass slides have less than 60% silicon dioxide on their surface. This inherently limits the amount of chemical bonding available on the surface. Porous material exhibits increased loading capacity of capture molecules. Typcal porous supports are gel pads, fused-fiber matrix, fibrous polymer matrix. The array can be constructed entirely of, the porous material, or can comprise a layer of porous material mounted on top of a flat surface such as glass, plastic, or metal.

In another embodiment capture molecules are present on different supports being preferentially beads with chemical or physical characteristics for their identification with a specific capture molecule. In still another embodiment, the support bears several microarrays separated by physical or chemical boundaries. In a preferred embodiment, the support has a multiwell format. Examples for physical barriers are wells, e.g. the support being a 96, 384, 1536 multi-well plate, thus creating separated localized areas onto which capture molecules maybe spotted individually. 384-well and 1536-well plates are available from BD Falcon for cell based assays (Merck Eurolab sa, Leuven, Belgium) or from Nunc A/S (Roskilde, Denmark). 6144 format microtiter plates are available from Parallel Synthesis Technologies Inc. (PSTI, Menlo Park, CA, USA). Other physical barriers are tubes such as 96, 384, 1536 or even 6144 tubes deposit at the surface of the support. Tubes are similar to the well formats but do not have a plain bottom sot that when deposit on the surface of the support, they create localized areas isolated from each other. An example for a chemical barrier is e.g described in DE 0019949735A1, where defined areas within a hydrophobic surface are provided with hydrophilic anchors allowing the precise location and confinement of capture molecules on a solid support.

The method of the invention can be performed using an insoluble solid support upon which are bound capture nucleotide sequences according to an array with a density of at least 10, 16, 20, 50, 100, 1000, 4000, 10 000 or more, different single stranded capture nucleotide sequences/cm² insoluble solid support surface.

Detectable labels suitable for use in the present invention include any composition detectable by chemical electrical or physical means preferably by electromagnetic light adsorption, diffraction, or emission. In an embodiment, the target molecules are labelled with a fluorescent dye. The fluorescent label can be incorporated into the target by enzymatic or chemical reaction. Typical enzyme reaction includes the incorporation of nucleotide analogues into the target. Alternatively, primers labelled at their 5' end with a fluorescent dye can be incorporated into the target. Fluorochromes can also be incorporated into the targets by chemical reaction such as the reaction of fluorescent dye bearing a N-hydroxysuccinimide (NHS) group with amines groups of the targets. Useful fluorescent dyes in the present invention include cyanine dyes (Cy3, Cy5, Cy7), fluorescein, texas red, rhodamine, green fluorescent protein. Patents teaching the use of such labels include U.S. Patent Nos. 3,817,837; 3,850,752; 3,939,350; 3,996,345; 4,277,437; 4,275,149; and 4,366,241. In a preferred embodiment, the fluorescent dye is cyanin 3.

In a particular embodiment, the target molecules bear a label which is recognised by a detectable molecule. Typical nucleotide label is the biotin which is recognised by streptavidin or antibiotin antibodies bearing a marker to be detected directly preferably a fluorescent dye or a nanoparticle or indirectly such as beads to be amplified as provided by the EU patent.

In a preferred embodiment, the method is used for the detection of several target molecules being present in concentrations between about 0.0001 and about 1000 nM in the detection solution and preferably between 0.001 and 10 nM.

In an embodiment, the method detects different targets having at least between 2 and 4 and better 5 log concentration difference. In a further embodiment, the method allows to quantify targets having at least 2 and better 5 and even better 6 log concentration differences.

In another preferred embodiment the signal values measured on the array for the different targets are proportional to the concentration of the targets in the solution. The concentration in the target is also preferably made in an absolute level by using internal standard processed as the sample nucleotide sequences to be measured.

In a preferred embodiment, several genes related to resistance to antibiotic(s) being expressed in a (micro) organism and being present in the same sample are simultaneously identified and/or quantified. The invention is particularly well adapted for the screening of a high number of different resistance genes.

In another preferred embodiment, at least 4 genes, preferably at least 10 genes and even 40 genes related to resistance to antibiotic(s) are simultaneously identified and/or quantified by the method of the invention. The genes related to resistance to antibiotic(s) are classified in 2 families being the genes specific for one antibiotic and genes common for several antibiotics. In a preferred embodiment, the genes specific for one antibiotic are among the genes presented in Tables I, II, III, V, VI and the genes common for several antibiotics and relating to general resistance mecanisms are among the genes presented in tables VII, VIII. More genes are possibly to be discovered or found to have similar fucntion related to the resistance to antibiotics. In another embodiment, at least one gene of each family is identified and/or quantified by the method of the invention.

In another embodiment, at least 6 genes specific for one antibiotic and at least 4 genes common for several antibiotics are simultaneously identified and/or quantified.

The kit according to the invention may also incorporate various media or devices for performing the method according to the invention. Said kit (or device) can also be included in an automatic apparatus such as a high throughput screening apparatus for the identification and/or the quantification of multiple genes related to resistance to antibiotics being expressed in a (micro) organism. Said kit or apparatus can be adapted for performing all the steps or only several specific steps of the method according to the invention.

In a specific embodiment, the invention is performed as proposed in the example 2. The detected resistance genes are related to the genes which confer a specific or a general mechanism of protection of the bacteria when in contact with the antibiotic. The protection is either total or partial and depends also of the time of contact and of the concentration used. The genes are either present in the bacteria and expressed or are present in the genome or in a plasmid and expressed under the contact with the antibiotic. In some case the gene are not present originally in the bacteria but are acquired by the bacteria, the most common mode of transmission being the transfer of a plasmid bearing the genetic information for the gene.
In particular, the specific antibiotic resistant genes are selected among the following classes.

For the MLS (Macrolide-Lincosamide-Streptogramin) resistance, the genes are related to the three different mechanisms of MLS resistance which have been found (Roberts, M.C et al) and are related to the antibiotic inhibition on the bacterial protein synthesis.

The first mechanism is an alteration of the target site. The antibiotic target modification is a postranscriptional methylation of the 23S rRNA by a adenine-N6-methyltransferase. In general, genes encoding these methylases have been designated erm (Erythromycin Ribosom Methylation). Several classes of rRNA methylases have been characterized. The table I resume the main classes of rRNA methylase genes involved in MLS resistance. These genes are generally located on plasmids or transposon.

The second mechanism of resistance if the more general system of efflux. A number of different antibiotic resistance genes code for transport (efflux) proteins. These proteins pump the antibiotic out of the cellular membrane, keeping intracellular concentrations low. All these proteins belong to two efflux proteins superfamily. The table VIII resumes the main efflux pumps. Many of these genes are located in plasmids or associated with conjugative elements in the chromosome. One group of genes are the Major facilitator superfamily (MFS) proteins. The *mef* genes have been found in majority in gram positive genera. Two mef genes have been characterized in the litterature : the *mef A* gene described in *Streptococcus Pyogenes* and the *mef E* found in *Streptococcus pneumoniae.* These two genes share 90% DNA and 91% amino acid homology and are grouped in a single class mef (A) gene. The second group is the ABC transporter superfamily. Several efflux pumps specific for MLS antibiotics belong to ABC transporter superfamily: the msr(A), *vga, vga(B)* genes from *Staphylococci* and the car (A), *ole* (B), *srm* (B) from *Steptomyces* genus.

The third mechanism of resistance is the inactivation of MLS antibiotics (Table VII). This mechanism of resistance is fairly rare in comparison with target site modification or efflux. There are several to inactivation mecanism :a) Degradation due to hydrolysis of the macrolide lactone ring by an esterase encoded by: the ere genes (Erythromycin resistance Esterase) founded in *E*. *coli,* S.aureus and *Pseudomonas sp. b*) Modification due to macrolide phosphorylation by a phosphotransferase encoded by *mph* genes ((Macrolide Phosphotransferase). c)Lincosamide nucleotidylation mediated by *lin* genes. d)The streptogramin B hydrolysis catalized by *vgb, gvb(B)* genes.

The Aminoglycosamides (gentamicin, Streptomycin , kanamycin etc) are commonly used for treatment of infections by both gram negative and gram positive bacteria (Shaw *et al, Davies et al*). These antibiotics bind to the ribosomes and thus interfere with protein synthesis. Resistance to aminoglycosides is due to enzymatic inactivation by acetyltransferase, nucleotidyltransferase and phosphotransferase. More than 50 aminoglycoside-modifying enzymes are already described associated generally with gram negative bacteria. See table II. Many of these genes encoding aminoglycoside-modifying enzymes are associated with plasmids and transposons.

The nomenclature used is defined as follows :
The terms AAC (acetyltransferase), ANT (nucleotidyltransferase or adenylyltransferase), APH (phosphotransferase) are used for the type of enzymatic modification. The predicted regiospecificity of group transfer is delineated by a number in parenthesis, the subfamily based on the aminoglycoside resistance profile is designated by a roman number and followed by a letter indicating a specific gene.

There is significant protein sequence homology among some of the aminoglycoside-modifiyng enzymes. Several distinct subfamilies could be identified :
the APH which included all of the 3'-phosphorylating enzymes already known. The AAC(3) cluster with all known AAC (3) enzymes and the APH (6)-I enzymes.

The glycopeptides are a third classes of antibiotics against which resistance have been developed (Jeljaszewicz et al). Vancomycin and teicoplanin are glycopeptide antibiotics of clinical interest. The mechanisme of antibacterial action depends on binding to the terminal D-alanyl-D-alanine of peptidoglycan precursors and inhibiting peptidoglycan synthesis. The Van resistance induces the synthesis of peptidoglycan precursors ending not in D-alanyl-D-alanine but in D-alanyl-D-lactate or D-alanyl-D-serine with reduced affinity to glycopeptides. Glycopeptide resistance requires the presence of three different enzymes : the ligases ( VanA, VanB, VanD...) catalysing synthesis of peptidoglycan precursor; the dehydrogenases (VanX, VanX_{B}, VanX_{D...}) generating D-lactate and the didepsipeptidases (VanH, VanH_{B}, VanH_{D}...) hydrolising normal pentapeptide precursor (Table III).
Genes encoding enzymes are grouped in clusters forming operons. 6 Van clusters are characterized. Three types of glycopeptide resistance, Van A, Van B, and Van D, result from the production of D-Ala-D-Lac terminating peptidoglycan precursors, whereas the VanC, VanE, and VanG types are characteriezd by the synthesis of precursors ending in D-Ala-D-Ser. Each of them have specific reponses to glycopeptides antibiotics (Table I).
The percent amino acid identity between each ligase is shown in table IV (McKessar *et al*). This shows a variability in the degree of similarity between each ligase (38 to 76 %).

Since few years, a glycopeptide resistance are been found in S.aureus. This mechanisme was presumably not related to vancomycin resistance in enterococci. The glycopepptide resistant phenotypes in Staphylococci are constitutive and are associated with the présence of a additional membrane proteinwich are identified as a D-hydroxy acid dehydrogenase encoded by the ddh gene (Boyle-Vavra. S et al).

Tetracyclines are broad-spectrum antimicrobial agents with activity against a wide range of bacteria. ( Robert M C. ) They act by binding to the 30S ribosomal subunit, resulting in the inhibition of protein synthesis. There have been 16 different tetracycline-resistant (Tet) determinants characterized.Two mechanisms of tetracycline resistance are known : the efflux system and the ribosomal protection.
The Efflux proteins have been the best studied. *tetA, tetB, tetC*, *tetD, tetE, tetG*, *tetH, tetI, tetK*, *tetL*, *tetA* (P) and otrB genes have been identified. All of these genes code for energy-dependent membrane-associated proteins which export tetracycline out of the cell. The efflux proteins confer resistance to tetracycline but not minocycline.

The tetracycline resistance can also result from the production of a protein that interacts with the ribosome such that protein synthetis is unaffected by the presence of the antibiotics. The determinants TetM, TetO, TetB(P), TetQ, TetS, TetT, TetW, OtrA confer resistance to tetracycline, doxycycline, amd minocycline. The distribtution of Tet resistance determinants among gram negative and gram positive species are shown in Table V The majority of the Tet determinants are frequently associated with either cojugative or mobilizable elements which may explain their wide distribution among bacterial species.

β-lactam antibiotics are among the most commonly used antimicrobial agents. They act on penicillin binding proteins (PBP) which are involved in cell wall synthesis ( Bush K.). Resistance is most often caused by the presence of β-lactamases, but mutations in PBPs resulting in reduced affinity for β-lacatam are already known.

Penicillins, cephalosporins, monobactams and carbapenems can all be hydrolyzed by multiple membres of β-lactamase family including cephalosporinases, metallo-β-lactamases, extended spectrum β-lactamases (ESBL). Up to now, at least 340 β-lactamases have been identified. All these enzymes are grouped on the basis of molecular structure. (table VI). From 1995 through 2000, 5 of the 11 subgroups of □-lactamases increased in number by at least 5.0%. Genes encoding β-lactamases can located either on plasmids or the bacterial chromosome and are found among both gram-negative and gram positive species.
Because of their increased spectrum of activity, enzymes of these family were called extended-spectrum β-lactamases (ESBL). ESBLs belong to a group of plasmid mediated serine β-lactamases (TEM, SHV, OXA...) . Today over 150 different ESBL have been described.

There are different types of ESBLs : 1. the TEM : more than 90 TEM-type β-lactamases are known, all derived from a parental sequence (TEM-1) They differ only a few amino acid substitutions. TEM is the most β-lactamase responsible for the amplicillin and penicillin resistance. 2. The SHV : There are now more than 25 SHV-type enzymes, all derived from SHV-1 and are few amino acid substitutions.The bla SHV-1 or related gene is integrated into bacterial chromosome. 3. The CTX-M : In recent years a new family of plasmid mediated ESBL called CTX-M that preferentially hydrolyse cetotaxime. They include the CTX-M type enzymes CTX-M-1 to CTXM-10. 4. The OXA : the OXA-type b-lactamases confer resistance to amplicillin and cephalothin and are a hight hydrolytic activity against axacillin and cloxacillin. The OXA-type ESBLs have been found mainly in P.aeruginosa. All variants ( OXA- 11, 13, 14, 15, 16, 17, 18, 19 and 28) have few amino acid substitutions.

Methicillin and its analogues are antibiotics which bind and inactive the PBPs involved in cell wall synthesis (Chambers H). High level resistance is dependent on the expression of an alternative PBP (PBP2a) encoded by the *mecA* gene. This gene is located on chromosome. His expression is constitutive or inducible by some □-lactam antibiotics.

Fluoroquinolone antibiotics exert their antibacterial effects by inhibition of the action of DNA gyrase and topoisomerase IV. DNA gyrase is a tetrameric enzyme composed of two A subunits and Two B subunits, encoded by gyrA and gyrB genes. The main function of this enzyme is to catalyse the negative supercoiling of DNA Topoisomerase IV is also a tetrameric enzyme composed of two A subunits and Two B subunits encoded by parC and parB genes. These subunit are highly homologous to gyrA and gyrB, ; the role of topoisomerase IV is associated with decatenating the daughter replicons.

Mechanismes of fluoroquinolone resistance include alterations in the drug target and alterations in the permeation of the drug to reach its target. No specific quinolone-modifying enzymes have been found as resistance mechanism.

The first one is the alterations in drug target enzyme. This mechanism appears to be the most dominant factors in expression of resistance to quinolones. In gram negative species the primary target of the quinolones seems to be the gyrase (Ruiz, J. 2003 Mechanisms of resistance to quinolones : target alterations, decreased accumulation and DNA gyrase protection). Journal of antimicrobial chemotherapy. 51, 1109-1117), whereas in gram positives species the primary target is topoisomerase IV (Hoopzer, D.C., 2001 Emerging mechanisms of fluoroquinolone resistance. Emerging infectious diseases. 7, 337-341). Sequence analysis of DNA from many bacterial species shows that resistance mutations tend to alter amino acids near the putative active site in the Gyr A protein. This region, extending between amino acids 67 and 106, is called the quinolone resistance-determining region (QRDR). To obtain high levels of resistance to fluoroquinolones, the presence of additional mutation(s) in gyr A and/or in another target is required. Mutations in gyrB subunit are much less common than those in GyrA. And are usually localized in a domain involved in interactions with the gyrA subunit.

The mutations described in the parC gene are also predominantly in the so-called quinolone-resistance determining region (QRDR). the most common substitutions occur at codons 80 and 84. The other substitutions in the same position have been found in gram positive bacteria. Up to known, only one substitution has been discribed in parE gene.

The second mechanism is the alterations that limits the permeation of drug to the target.
Decreased quinolone uptake can be due to two factors : an increase in the bacterial impermeability to these antibacterial agent or the overexpression of efflux pumps. Quinolones may cross the outer membrane through specific porins or by diffusion through the phopolipid bilayer. Thus alterations in the composition of porin and/or in the lipopolysaccharides may alter the suseptibility to quinolones. In ompF porin and lipopolysaccharides defective mutants, increased susceptibility to quinolones has been discribed (Hirai K. et al 1986 Differences in susceptibility to quinolones of outer membrane mutants of Salmonella typhimurium and E.coli. Antimicrobial agents and chemoterapy. 535-538.)

Beside the mechanisms of resistance which are more or less specific of the antibiotic classes, the bacteria provides some systems of defense which are common to many different bacteria and apply to many antibiotics and in this way would be considered as non specific mechanisms. The main one is the antibiotic efflux pumps (Van Bambeke et al. 2003, Journal of Antimicrobial Chemotherapy 51, 1055-1065; Webber, M A. and Piddock J.V., 2003, Journal of antimicrobial chemotherapy 51, 9-11). This mechanism involves the active extrusion of antimicrobials from the cell by drug-transport systems. These transporters are classified as SDR transporters : Specific Drug Resistance transporters (like tertracycline efflux proteins) or as MDR transporters : MultiDrug Resistance transporters (can transport a wide variety of structurally unrelated compounds). The multidrug transporters include as primary active transporters the ABC-transporters and as secondary active transporters the SMR, MATE,RND and the MFS.( Lage et al H., 2003, International journal of antimicrobial agent 22, 188-199).

In the prokaryotic kingdom there are five major families of efflux transporters classified in two groups: The first group is composed of primary active transporters that are energized by ATP hydrolysis. These primary transporters are represented by the superfamily of ATP-binding cassette (ABC) transporters. The second group consists of secondary active transporters that mediate the drug efflux reaction in a coupled exchange with proton or sodium ions along a concentration gradient as symport or antiport. Members of this group are: - the Major Facilitator Superfamily (MFS) - the Small Multidrug Resistance Superfamily (SMR) -the Resistance Nodulation cell Division superfamily (RND) - the Mutidrug And Toxic compound Extrusion superfamily (MATE).
In prokaryotic organism the secondary active multidrug transporters are predominantly active in drug extrusion. Only few ABC transporters are identified mainly in gram positive bacteria (MsrA protein from S.epidermidis and S.aureus, LmrA protein in L.lactis)(Lage et al H., 2003, International journal of antimicrobial agent 22, 188-199). Several transporters were identified for resistance mechanism towards various classes of antibiotics :
macrolides, fluoroquinolones, B-lactams and aminoglycosides.

The table VIII shows the antibiotics transporters identified in some important pathogens and the antibiotics which are drived (Van Bambeke et al. 2000, Pharmacology, 60 :457-470. and Van Bambeke et al. 2003, Journal of Antimicrobial Chemotherapy 51, 1055-1065). We can see that :1) Several transporters recognize very different classes of antibiotics, especially in resistance nodulation division superfamily (NDR). This may explain a cross resistance of several bacteria to structurally unrelated drugs. 2) Given species may express quite different drug transporters, leading again to multiresistant phenotypes. Efflux may also cooperate with other resistance mechanism to confer not only high level but also broadspectrum resistance. For example in E.coli the expression of class C β-lactamases which confer resistance to first and second- generation of cephalosporins and the expression of pump AcrB confers resistance to most penicillins. 3) A given antibiotic may be recognized by different pumps.
Most of genetic elements encoding efflux pumps are located on plasmids or on conjugative or transformable transposons located either on plasmid or in the chromosome. Thus resistance by efflux can be easily disseminated.

Although most prokaryotic drug transporters belong to the class of secondary active transporters, several drug transporting systems use the energy of ATP hydrolysis to drive drug extrusion and belong to the ABC-transporter family. Most drug transporters mediate single drug resistance (SDR) in gram positives bacteria : the MsrA protein from S.epidermidis and S.aureus and the Ard1 protein from S.capreolus. Several MDR transporters are also identified (LmrA in L.lactis, HorA in L.brevis). No biological active SDR or MDR ABC-transporter has yet been identified in gram negative bacteria. (Lage H., 2003, International journal of antimicrobial agent 22, 188-199).

In fungi, two major classes of xenobiotics transporters are involved in drug resistance , the family of proton-motive force driven MFS-transporters and the family of primary active ABC-transporters. C.albicans wich has become increasing clinical problem has five genes encoding members of family of ABC-transporters CDR1, CDR2, CDR3, CDR4 and CDR5. The CDR1 and CDR2 proteins play a major role in drug resistance.

The present invention will be described in details in the following non-limiting examples in reference to the enclosed figures.

### Examples

### Example 1

### Preparation of the microarray of the invention

### Capture nucleotide sequence and immobilisation

The protocol described by Schena et al (Proc. Natl Acad. Sci. USA 93, 10614 (1996)) was followed for the grafting of aminated DNA to aldehyde derivatised glass. The aminated capture nucleotide sequences were spotted from solutions at concentrations of 400 nM. The capture nucleotide sequences were printed onto microscopic glass slides with a home made robotic device (125 *µ*m pins from Genetix (UK)). The Diaglass (aldehyde) microscope slides were from Eppendorf (Hamburg, Germany). The spots have 250 *µ*m in diameter and the volume dispensed is about 0,5 nl. Slides were dried at room temperature and stored at 4 °C until used.
The capture probes were long aminated nucleotides of more than 300 bases complementary of the target nucleotide sequences. They are anti-sense oriented as compared to the mRNA strand.
The array (figure 1) comprises 206 probes specific for the resistance genes and 54 controls (amplification, positive and negative hybridization, positive and negative detection controls). Positive hybridization controls are capture nucleotide sequences that hybridized with labelled targets added to the hybridization mixture and negative controls are capture nucleotide sequences unrelated to the target sequences.
The array as presented in figure 1 has been designed for detection of homologous sequences which have less than 80% homology. Some capture probes are specific for one family member and other are consensus for several family members when the homology is too high.
Additional probes may be added to the array if one want to discriminate between sequences with higher homology than 80%. In this case, small capture sequences specific for the target have to be used, in the range of 15 to 30 bases. They are inserted on the top of a spacer which has the following sequence:
GAATTCAAAGTTGCTGAGAATAGTTCAATGGAAGGAAGCGTCTTCTTAAAATCTAAAGA A. The spacer in aminated and is fixed on the support by its 5' end.

### Example 2

### Detection of resistance related gene from bacteria on chips Using IVT amplification

The bacteria were obtained from cultures and were treated in the following way.

### 1.Total RNA isolation

Total RNA is isolated using the Ambion RNA Isolation Kit RiboPure™-Bacteria (Cat #1925).

### 2. mRNA purification of bacteria

This step is performed using Ambion's MICROBExpress Kit. Magnetic beads derivatized with capture oligonucleotides capture and remove abundant bacterial rRNA (16S rRNA and 23S rRNA) from total bacteria RNA mixture.

### Anneal RNA and Capture Oligonucleotide Mix

- Add 2-10 *µ*g total RNA to 200 *µ*l Binding Buffer
- Add 4 *µ*l Capture OligoMix : Add 4 *µ*l of Capture Oligo Mix to the RNA in Binding Buffer.Close the tube and tap or vortex gently to mix, and microfuge briefly to get the mixture to the bottom of the tube. Heat to 70°C for 10 min.
- Anneal at 37°C for 15 min to 1 hour. The incubation allows the capture oligonucleotides to hybridize to homologous regions of the 16S and 23S rRNAs.

### Capture the rRNA and Recover the Enriched mRNA

- Heat the Wash Solution to 37°C
- Add 50 *µ*l prepared Oligo MagBeads to the RNA/Capture Oligo
- Mix and incubate at 37°C for 15 min
- Capture the Oligo MagBeads, and move the mRNA to a Collection Tube
- Recover any remaining mRNA from the Oligo MagBeads by washing them with 100 *µ*l Wash Solution at 37°C and recovering the wash

### Precipitate and resuspend the Enriched mRNA

- Add the following to the pooled mRNA from (the volume should be ~350 µl), and briefly vortex to mix : 1^{~}10th volume 3 M Sodium Acetate (35 µl); 1^{~}50th volume Glycogen .(5 mg/ml), the final concentration will be 100 µg/ml (7 µl)
- Add 3 volumes ice cold 100% ethanol (1175 µl) , and vortex to mix thoroughly.
- Precipitate at -20°C for at least 1 hr.
- Centrifuge for 30 min at 10,000 X g (typically ~13,000 rpm in a microcentrifuge) and carefully decant and discard the supernatant.
- Do a 70% ethanol wash as follows: add 750 *µ*l ice cold 70% ethanol and vortex briefly, centrifuge for 5 min at 10,000 X g. Discard the supernatant.
- Do a second 70% ethanol wash.
- Briefly re-spin the tube after discarding the second 70% ethanol wash. Carefully remove any remaining supernatant with a pipettor, being careful not to dislodge the pellet.
- Air dry the pellet for 5 min. Do not air dry the pellet for more than 5 min.

### Resuspend the enriched mRNA in an appropriate buffer

- Resuspend the RNA pellet in 25 µl TE (10 mM Tris-HCl pH 8, 1 mM EDTA) .
- Rehydrate the RNA for 15 min at room temperature. Vortex the sample vigorously if necessary to resuspend the RNA. Collect the sample by brief centrifugation. If the pellet will not go into solution after 15 min at room temp and vigorous vortexing, heat the sample to 70°C for 5 min.
Evaluate rRNA removal with the RNA 6000 Nano LabChip® Kit.

### 3. IVT Amplification of RNAm from bacteria

This step is performed using Ambion's MessageAmp II-Bacteria Kit. This kit is developed to facilitate whole genome expression analysis from bacterial samples. The kit is a linear in vitro transcription (IVT)-based RNA amplification system (Philips and Eberwine, 1996 Methods in Enzymology, 10: 283-288) to produce amplified antisense RNA (aRNA also called copied RNA or cRNA).
The first step in the procedure is polyadenylation using *E.coli* Poly(A) Polymerase (PAP) which ensures polyadenylation of bacterial RNA molecules.

### Polyadenylation of Template RNA

- Bring RNA samples to 5 *µ*l with Nuclease-free Water: place up to 1000 ng of Total RNA (typically 100-500 ng) or up to 500 ng mRNA (typically 10 ng-200 ng) into a sterile RNase-free microfuge tube; add Nuclease-free Water to bring each sample to 5 *µ*l. Vortex briefly to mix, then centrifuge to collect sample at the bottom of the tube.
- Incubate 10 min at 70°C, then place on ice for 3 min : incubate 10 min at 70°C, preferably in a thermal cycler; remove the RNA samples from the 70°C incubator and centrifuge briefly (∼5 sec) to collect sample at the bottom of the tube. Place the mixture on ice for 3 min.
- Add 5 µl of Polyadenylation Master Mix and place at 37°C : at room temp assemble the Polyadenylation Master Mix in the order shown: Amount Component (1.5 µl Nuclease-Free Water, 1.0 µl 10X Poly(A) Tailing Buffer, 1.0 µl RNase Inhibitor, 0.5 µl Poly (A) Tailing ATP, 1.0 µl PAP MessageAmp™ II-Bacteria Kit). Gently vortex to make a homogenous mixture without inactivating the enzyme, then centrifuge for ~5 sec to collect the master mix at the bottom of the tube. Transfer 5 *µ*l of Polyadenylation Master Mix to each RNA sample, mix thoroughly by gentle vortexing and follow with a quick spin to collect the reaction. Place the samples in a 37°C incubator.
- Incubate for 15 min at 37°C
- Incubate reactions for 15 min at 37°C, then centrifuge briefly to collect the reaction at the bottom of the tube.
- Place reactions on ice and proceed to the next step
- After the 37°C incubation, place the reactions on ice and proceed immediately to the reverse transcription.

### Reverse Transcription to Synthesize First Strand cDNA

In this step, the tailed RNA is reverse transcribed in a reaction primed with an unique oligo(dT) primer bearing a T7 promoter. The kit employs ArrayScript™, a reverse transcriptase engineered to produce higher yields of first strand cDNA than wild type enzymes. ArrayScript catalyses the synthesis of virtually full-length cDNA, which is the best way to ensure production of reproducible microarray samples.
- Prepare Reverse Transcription Master Mix : 3 µl Nuclease-free Water, 1 µl T7 Oligo(dT) VN, 1 µl 10X First Strand Buffer, 4 µl dNTP Mix, 1 µl ArrayScript.
- Gently vortex to make a homogenous mixture without inactivating the enzyme, then centrifuge for ~5 sec to collect the master mix at the bottom of the tube.
- transfer 10 *µ*l of Reverse Transcription Master Mix to each sample, mix thoroughly by gentle vortexing, and follow with a quick spin to collect the reaction; place the samples in a 42°C incubator.
- Incubate for 2 hr at 42°C, then centrifuge briefly (∼5 sec) to collect the reaction at the bottom of the tube.
- Place the tubes on ice and immediately proceed to the second strand cDNA synthesis.

### Second Strand cDNA Synthesis Bacterial RNA Amplification Protocol

This step converts the single-stranded cDNA with the T7 promoter primer into double stranded DNA (dsDNA) template for transcription. The reaction employs DNA polymerase and Rnase H to simultaneously degrade the RNA and sythesize second strand cDNA.
- Add 80 *µ*l Second Strand Master Mix to each sample. On ice, prepare a Second Strand Master Mix by mixing the following reagents in the order listed below. Amount Component (63 *µ*l Nuclease-free Water, 10 *µ*l 10X Second Strand Buffer, 4 *µ*l dNTP Mix, 2 *µ*l DNA Polymerase, 1 *µ*l RNase H); gently vortex to make a homogenous mixture without inactivating the enzymes, then centrifuge for ~5 sec to collect the master mix at the bottom of the tube; transfer 80 *µ*l of Second Strand Master Mix to each sample, mix thoroughly by gentle vortexing, and follow with a quick spin to collect the reaction; place the samples in a 16°C thermal cycler.
- Incubate 2 hr in a 16°C thermal cycler.
- Place reactions on ice.

### cDNA Purification

This step removes RNA, primers, enzymes and salts from the dsDNA that inhibit in vitro transcription.
- Before beginning the cDNA purification, preheat the 10 ml bottle of Nuclease-free Water to 50°C for at least 10 min.
- Add 250 *µ*l of cDNA Binding Buffer to each sample and mix thoroughly by gently vortexing.
- Pass mixture through a cDNA Filter Cartridge : Pipet the cDNA sample\cDNA Binding Buffer onto the center of the cDNA Filter Cartridge; centrifuge for -1 min at 10,000 x g, or until the mixture is through the filter; discard the flow-through and replace the cDNA Filter Cartridge in the wash tube: wash with 500 *µ*l Wash Buffer; make sure that the ethanol has been added to the bottle of Wash Buffer before using it; Apply 500 *µ*l Wash Buffer to each cDNA Filter Cartridge; centrifuge for 1 min at 10,000 x g, or until all the Wash Buffer is through the filter; discard the flow-through and spin the cDNA Filter Cartridge for an additional minute to remove trace amounts of ethanol; transfer cDNA Filter Cartridge to a cDNA Elution Tube.
- Elute cDNA with 20 *µ*l 50°C Nuclease-free Water: to the center of the filter in the cDNA Filter Cartridge, apply 2 x 10 *µ*l of preheated (50°C) Nuclease-free Water: leave at room temperature for 2 min and then centrifuge for -1.5 min at 10,000 x g, or until all the Nuclease-free Water is through the filter. The double-stranded cDNA will now be in the eluate (~16 µl).
- The purified cDNA can be stored overnight at -20°C at this point if desired.

### In Vitro Transcription to Synthesize aRNA

The resulting cDNA is then transcribed with T7 RNA polymerase to generate some hundreds to thousands of antisense RNA (aRNA) copies of each RNA in the sample.
- At room temperature, assemble an IVT Master Mix by adding reagents in the following order : 4 µl T7 ATP Soln (75mM), 4 µl T7 CTP Soln (75mM), 4 µl T7 GTP Soln (75mM), 4 *µ*l T7 UTP Soln (75mM), 4 *µ*l 10X T7 Reaction Buffer, 4 *µ*l T7 Enzyme Mix.
- Mix well by gently vortexing. Centrifuge briefly (~5 sec) to collect the IVT Master Mix at the bottom of the tube and place on ice.
- Transfer 24 µl of IVT Master Mix to each sample containing ~16 µl double stranded cDNA obtained in the previous step, mix thoroughly by gentle vortexing, centrifuge briefly to collect the reaction, and place the tubes in a 37°C incubator.
- Incubate for 14 hr at 37°C
- Add DNaseI to the reaction and incubate further at 37°C for 15 min.
- Mix thoroughly by gentle vortexing.
- Place the diluted aRNA on ice if the aRNA purification step will be done immediately. Alternatively, the aRNA can be stored at -20°C.

### aRNA Purification

This purification removes enzymes, salts, and unincorporated nucleotides from the aRNA. At the end of the purification, the aRNA can be eluted from the filter with Nuclease-free water.
- Preheat Nuclease-free Water to 50-60°C (≥10 min)
- Add 350 *µ*l aRNA Binding Buffer and mix
- Add 250 *µ*l 100% ethanol and pipette 3 times to mix
- Pass samples through an aRNA Filter Cartridge(s)
- Wash with 650 µl Wash Buffer. Make sure that the ethanol has been added to the bottle of Wash Buffer before using it.
- Elute aRNA with 2 x 50 *µ*l preheated Nuclease-free Water
- Concentrate if necessary and assess the aRNA yield
- Store aRNA at -70°C under aliquots of 5-20 *µ*g.

### Copy and labelling of aRNA

The aRNA were then copied into DNA in order to avoid possible degradation of the RNA by non specific RNAse during the long hybridization period. Five *µ*g of aRNA are first mixed with 3 *µ*g of random primer in 7,5 *µ*l of RNase-free water and 2 *µ*l of a solution of 6 different synthetic well-defined poly(A+) RNAs. These latter served as internal standards to assist in quantification and estimation of experimental variation introduced during the subsequent steps of analysis. RNA/primer mix is incubated at 70°C for 10 min and cooled on ice for 5 min. The following reagents are added: 4 *µ*l of 5X first strand buffer, 2 *µ*l 0.1 M DTT, 2 *µ*l 10X dNTP mix(5 *µ*M dATP, dTTP, dGTP and 0.8 *µ*M dCTP, 0.8 *µ*M biotin-dCTP) and 1.5 *µ*l Superscript™ II (200 U/*µ*l). The labelling reaction is carried out at 42°C for 3 hour during which 1,5 *µ*l Superscript™ II is added to the reaction at the end of the 90 minutes. The input RNA is removed by RNAse H treatment (2U during 20 min. at 37°C). The RT product is purified in a Microcon® YM-30 column (Millipore).

### 4. Hybridization of biotinylated cDNA:

The microarray used in this study is prepared according to example 1. It is composed of 206 capture probes representative of the resistance related gene for specific antibiotic and for common antibiotics as provided in tables I to VIII. The composition of the chip is presented in figure 1. Each capture molecule for the gene is present in triplicate. The arrays also contain several different controls including positive and negative detection control, positive and negative hybridization control, amplification control. In this example each spots was covered with a capture probe being a polynucleotide species, which allows the specific binding of one or several target polynucleotide(s) belonging to the same gene family. All sequences have been designed to be gene specific and have been prepared using bacteria cDNA clones.
Hybridization chambers were from Biozym (Landgraaf, The Netherlands). Hybridization mixture consisted in biotinylated cDNA (the total amount of labelled cDNA), 6.5 µl HybriBuffer A (Eppendorf, Hambourg, Germany), 26 µl HybriBuffer B (Eppendorf, Hambourg, Germany), 8 µl H₂O and 2 µl of positive hybridization control. Hybridization was carried out overnight at 60°C. The micro-arrays were then washed 4 times for 2 min with washing buffer (Eppendorf, Hamburg, Germany).

### 5. Fluorescent detection

The micro-arrays were than incubated for 45 min at room temperature with the Cy3-conjugated IgG Anti biotin (Jackson Immuno Research laboratories, Inc #200-162-096) diluted 1/1000 X Conjugate-Cy3 in the blocking reagent and protect from light.
The micro-arrays were washed again 4 times for 2 minutes with washing buffer and 2 times for 2 minutes with distilled water before being dried under a flux of N₂.
The hybridized micro-arrays were scanned using a laser confocal scanner "ScanArray" (Packard, USA) at a resolution of 10 *µ*m. To maximize the dynamic range of the assay the same arrays were scanned at different photomultiplier tube (PMT) settings. After image acquisition, the scanned 16-bit image were imported to the software, 'Imagene4.0' (Biodiscovery, Ca, USA), which was used to quantify the signal intensities. Data mining and determination of significantly expressed gene were determined with specific software.

### Example 3

### Detection of resistance related gene from bacteria on chips using PCR amplification

As an alternative to IVT Amplification, cDNA can also be used as template for PCR amplification. Isolation of bacterial mRNA and their polyadenylation were performed as in example 2 points 1, 2 and part of 3. The following steps are performed after the polyadenylation.

### 1. Reverse transcription of bacterial mRNA

1 µl of poly(A+) RNA sample (0.5 µg/µl) was mixed with 2 µl oligo (dT) 12-18 (0.5 µg/µl, Roche), 5.5 µl H2O. After an incubation of 10 minutes at 70°C and 5 minutes on ice, 9 µl of reaction mix were added. Reaction mix consisted in 4 µl Reverse Transcription Buffer 5X (Gibco BRL), 1 µl RNAsin Ribonuclease Inhibitor (40 U/ml, Promega), and 2 µl of a 10X dNTP mix, made of dATP, dTTP, dGTP, dCTP (5 mM each, Roche) .

After 5 min at room temperature, 1.5 µl SuperScript II (200 U/ml, Gibco BRL) was added and incubation was performed at 42°C for 90 minutes. Addition of SuperScript and incubation were repeated once. The mixture was then placed at 70°C for 15 minutes and 1 µl Ribonuclease H (2U/µl) was added for 20 minutes at 37°C. Finally, a 3-minutes denaturation step was performed at 95°C. The cDNA, was kept at -20°C. Purify the cDNA using the Rneasy kit (Qiagen).

### 2. Elongation of Template cDNA

Terminal transferase catalyses the addition of dNTPs to the 3' terminus of single stranded DNA. This enzyme is used here to add a poly(dC) tail to the 3' end of cDNA. This tail will be used as template for oligo(dG) primer annealing.
- Add to the template cDNA, 4 *µ*l 5x Tdt Reaction buffer, 4 *µ*l of CoCl2 25 mM, 1 *µ*l dCTP 10 mM, 1 *µ*l terminal transferase rec.(Roche, cat N°3333566)
- Add double distilled water to a final volume of 20 *µ*l.
- Mix and centrifuge briefly.
- Incubate at 37°C for 15 min.
- Place on ice.
- Stop the reaction by adding 2 *µ*l 0.2M EDTA (pH 8.0).

The synthesized single strand cDNA comprises a poly (dT) tail at the 5' end and a poly(dC) at the 3' end. The length of the elongated poly(dC) fragment is preferably 20-40 bases.

### 3. PCR amplification

The two primers used for PCR amplification are Oligo(dT) 30-mer and Oligo(dG) 15-mer.
The PCR was performed in a final volume of 50 *µ*l containing: 2 mM MgCl₂, 10 mM Tris pH 8.4, 50 mM KCl, 1 µM of each primer, 200 µM of dATP, 200 µM of dCTP, 200 µM of dGTP, 400 µM of dUTP, 10 *µ*M of biotin-11-dATP, 10 *µ*M of biotin-11-dCTP, 1.5 U of Taq DNA polymerase Ultratools,1U of UNG (uracyl N-glycosylase) , and the template single strand cDNA. Samples were first denatured at 94 °C for 3 min. Then 40 cycles of amplification were performed consisting of 30 sec at 94 °C, 30 sec at 55 °C and 30 sec at 72 °C and a final extension step of 10 min at 72 °C. Water controls were used as negative controls of the amplification.

### 4. Hybridization of biotinylated amplicons on microarray

At 65 *µ*l of hybridisation solution containing 2 nM positive hybridization control (Eppendorf, Hamburg, Germany) were added 5 *µ*l of amplicons and the solution was loaded on the array framed by an hybridisation chamber. The hybridisation was carried out at 60° for 2 h. Samples were washed 4 times with a washing buffer.

### 5. Colorimetric detection

The glass samples were incubated 45 min at room temperature with colloidal gold-conjugated IgG Anti biotin 1000 x diluted in blocking buffer (Eppendorf, Hamburg, Germany). After 5 washes with washing buffer, the presence of gold served for catalysis of silver reduction using a staining revelation solution (Eppendorf, Hamburg, Germany). The slides were incubated 3 times 10 min with the revelation mixture, then rinsed with water, dried and analysed using a microarray reader. Each slide was then quantified by a specific quantification software.

Table I. Resistance related Gene for Specific antibiotic: Macrolide-Lincosamide-Streptogramin. There will be a capture probes for each gene of the mRNA methylase enzymes

Table II. Resistance related Gene for Specific antibiotic: Aminoglycoside resistance. The table gives the capture probes provided for the different genes.

Table III. Resistance related Gene for Specific antibiotic: Glycopeptides resistance. A capture probe will be provided for each gene.

Table IV Identity of amino acid sequence between the different enterococcal ligases .

Table V. Resistance related Gene for Specific antibiotic: -Tetracycline Resistance. There is a capture probe for each gene.

Table VI. Resistance related Gene for Specific antibiotic: β-lactam antibiotic resistance. The table gives the capture probes provided for the different genes.

Table VII: Resistance related Gene for different antibiotic: the inactivation enzymes. A capture probe will be provided for each gene.

Table VIII The main efflux tranporters for the antibiotic substrates. The capture probes will be provided against each of the transporters.

**Table I Resistance related Gene for Specific antibiotic: Macrolide-Lincosamide-Streptogramin. There will be a capture probes for each gene of the mRNA methylase enzymes**

| **Class** | **Protein** | **Gene name (= probe name)** |
|---|---|---|
| A | Erm (A) | erm (A) |
| B | Erm (B) | erm (B) |
| C | Erm (C) | erm (C) |
| D^{d} | Erm (D) | erm (D) |
| E | Erm (E) | erm (E) |
| F | Erm (F) | erm (F) |
| G | Erm (G) | erm (G) |
| H^{b} | Erm (H) | erm (H) |
| I^{b} | Erm (I) | erm (I) |
| N^{b} | Erm (N) | erm (N) |
| O^{b} | Erm (O) | erm (O) |
| Q | Erm (Q) | erm (Q) |
| R | Erm (R) | erm (R) |
| S^{b} | Erm (S) | erm (S) |
| T^{b} | Erm (T) | erm (T) |
| U^{b} | Erm (U) | erm (U) |
| V^{b} | Erm (V) | erm (V) |
| W^{b} | Erm (W) | erm (W) |
| X^{b} | Erm (X) | erm (X) |
| Y^{b} | Erm (Y) | erm (Y) |
| Z^{b} | Erm (Z) | erm (Z) |

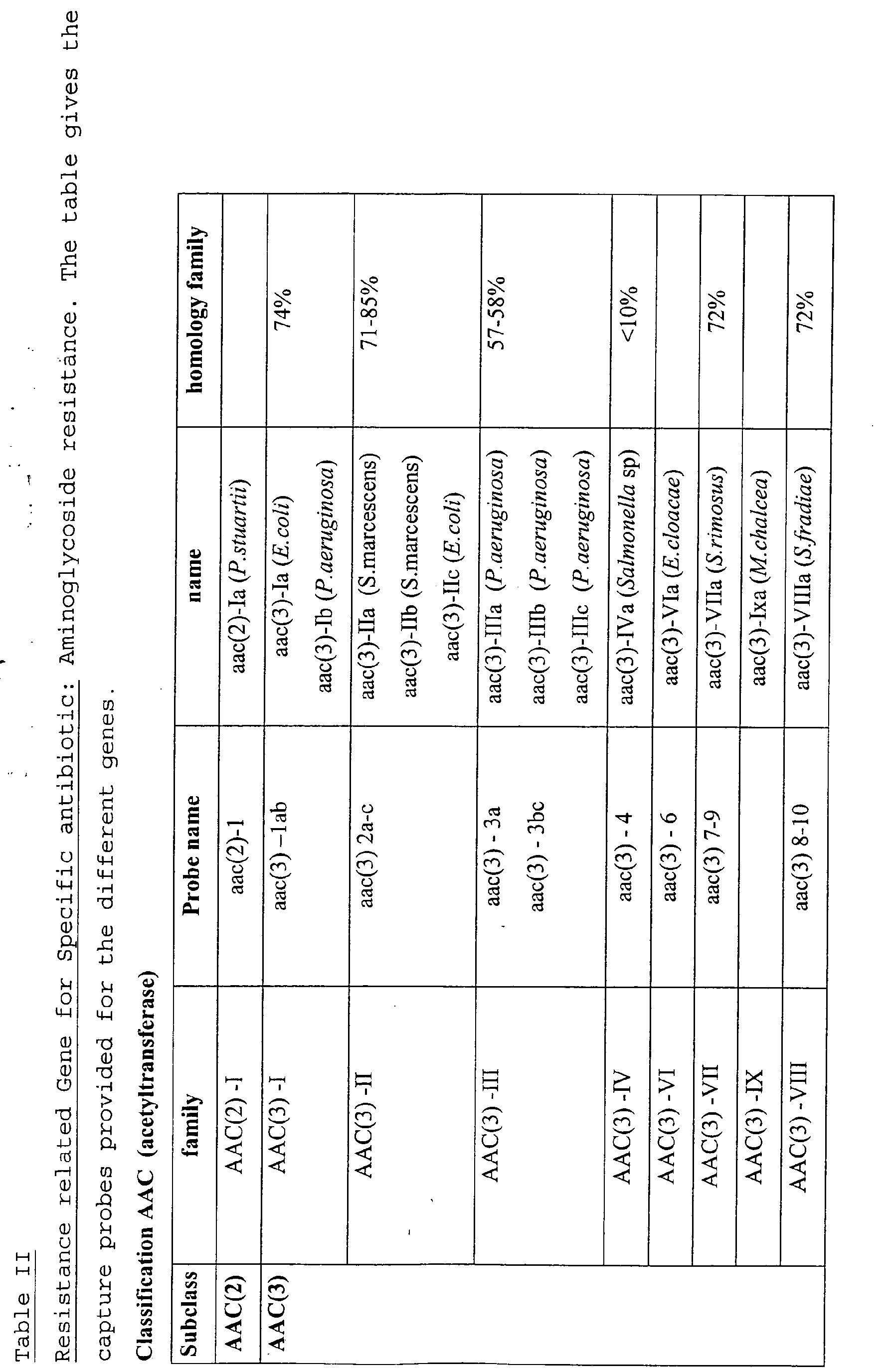

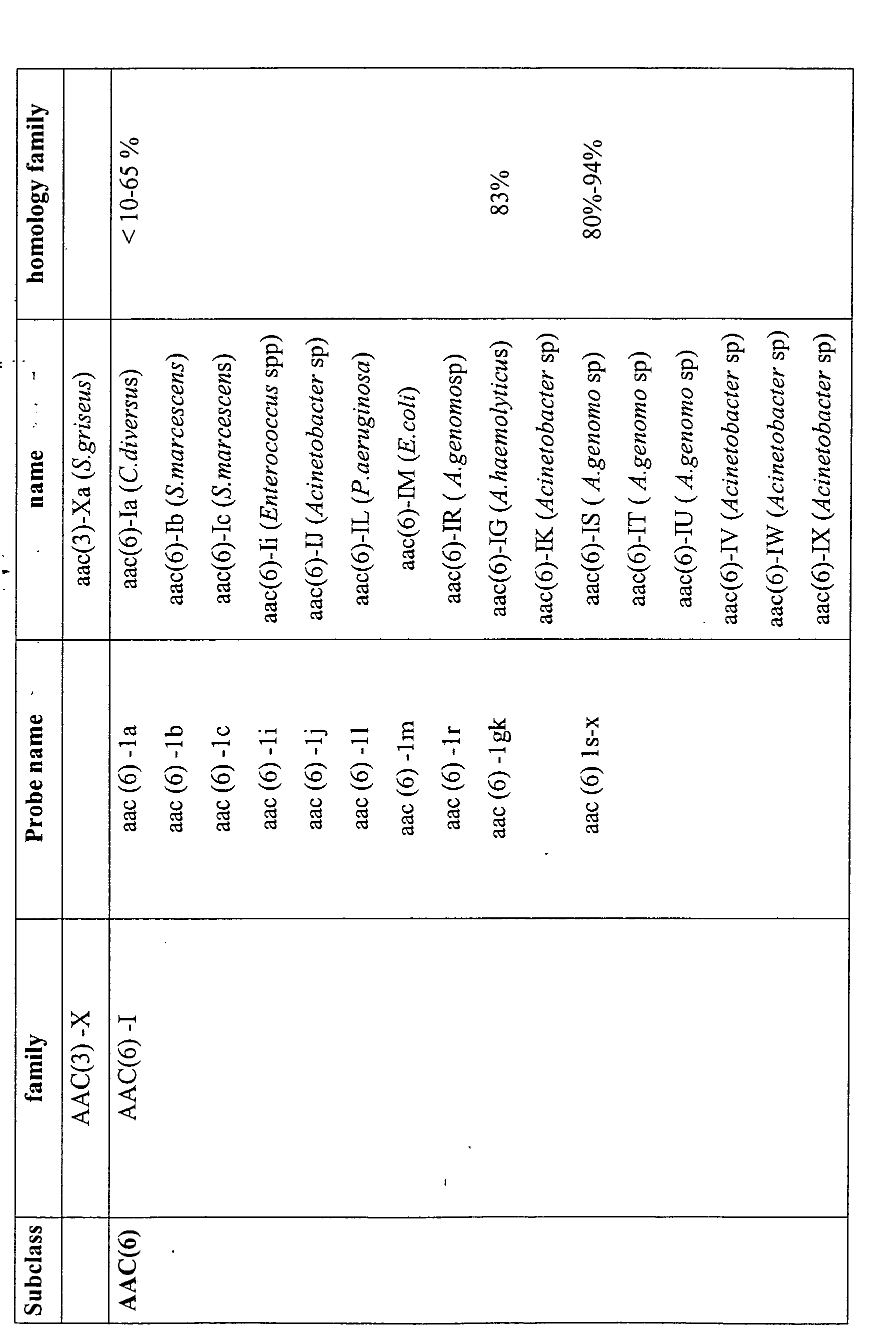

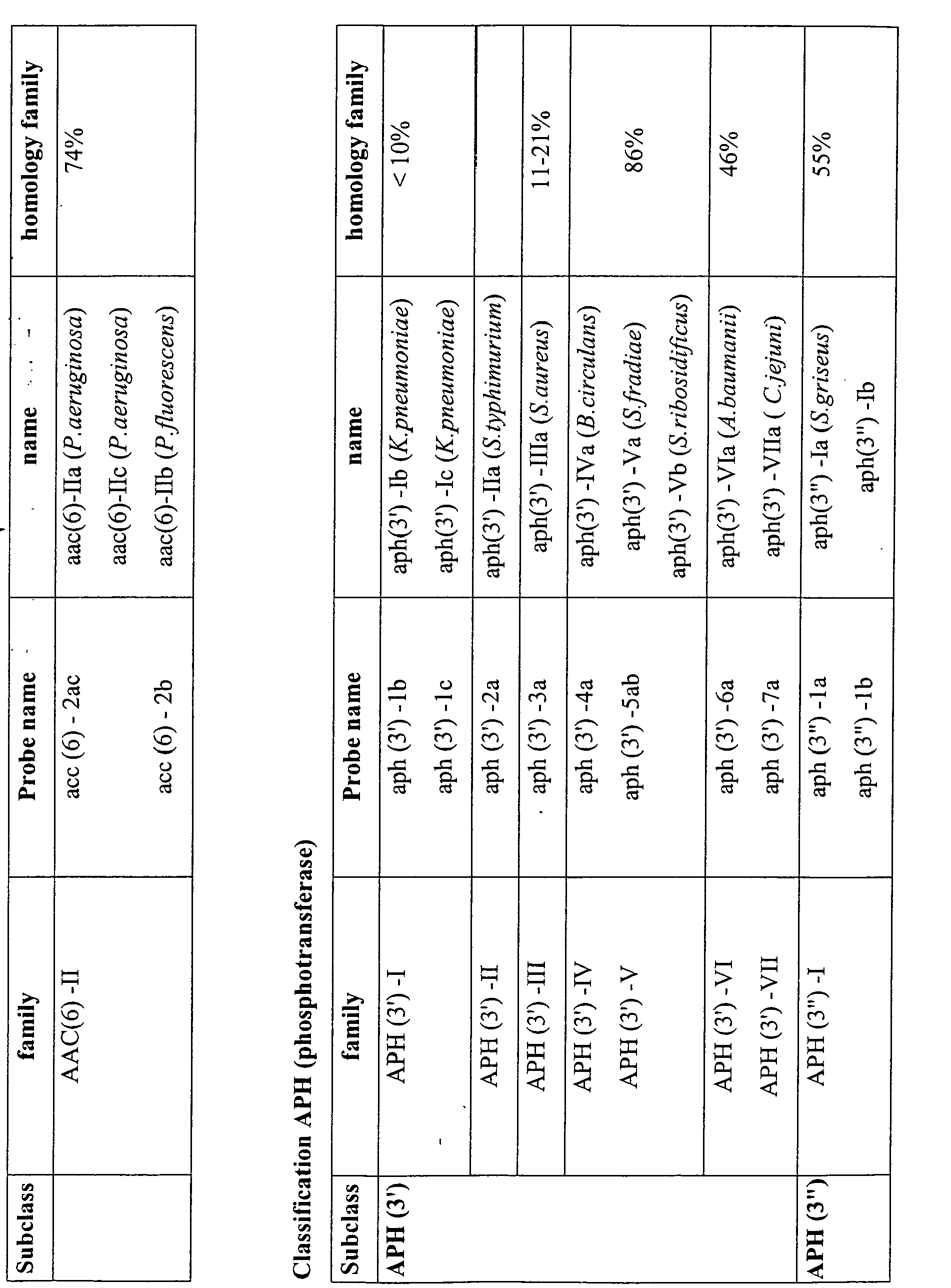

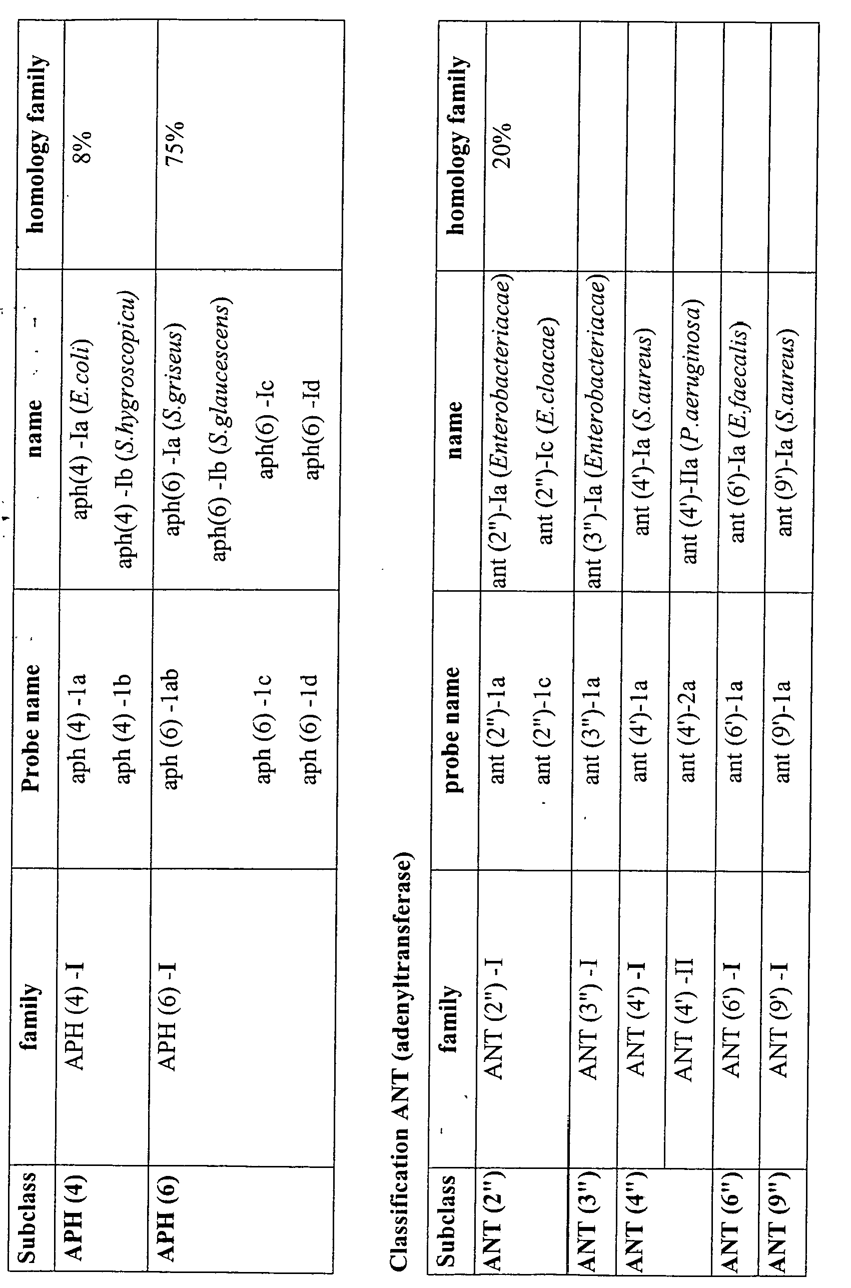

**Table III Resistance related Gene for Specific antibiotic: Glycopeptides resistance Ligases enzymes**

| **Gene** | **Name** |
|---|---|
| Van | A |
| Van | B |
| Van | C |
| Van | D |
| Van | E |
| Van | G |

**Table IV**

| sequence compared | % sequence identity (no.of overlapping amino acids) | | | | |
|---|---|---|---|---|---|
| | **van G** | **Van A** | **Van B** | **VanC** | **VanD** |
| **VanA** | 44 (351) | | | | |
| **VanB** | 47 (354) | 76 (341) | | | |
| **VanC** | 42 (347) | 38 (349) | 39 (350) | | |
| **VanD** | 46 (347) | 69 (342) | 67 (343) | 38 (350) | |
| **VanE** | 39(201) | 44 (206) | 42 (206) | 54 (202) | 44 (206) |

**Table V Resistance related Gene for Specific antibiotic: Tetracycline Resistance Tet proteins of efflux for Tetracycline**

| **Gene Tet** |
|---|
| A |
| B |
| C |
| D |
| E |
| G |
| H |
| I |
| K |
| L |
| M |
| O |
| P |
| Q |

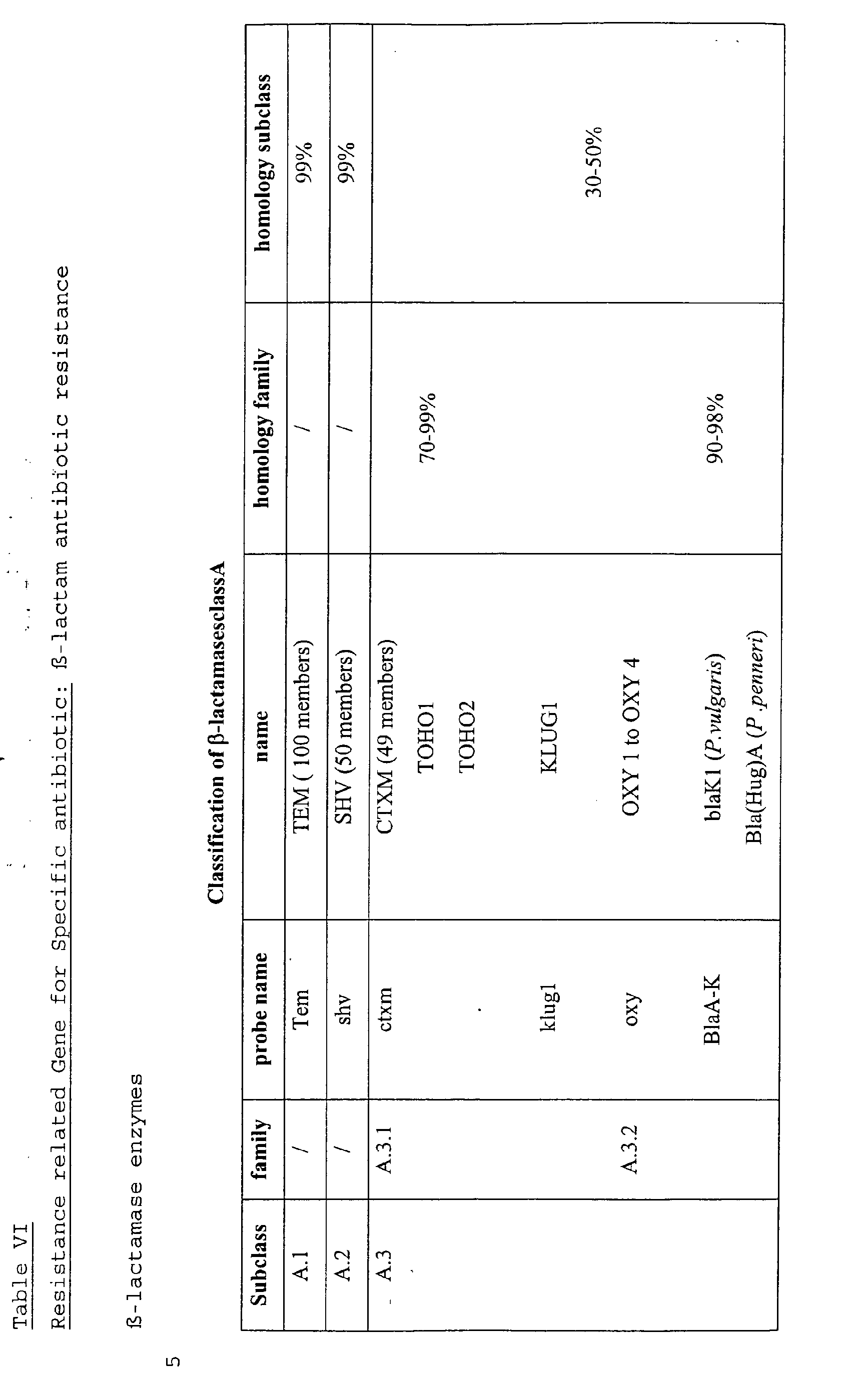

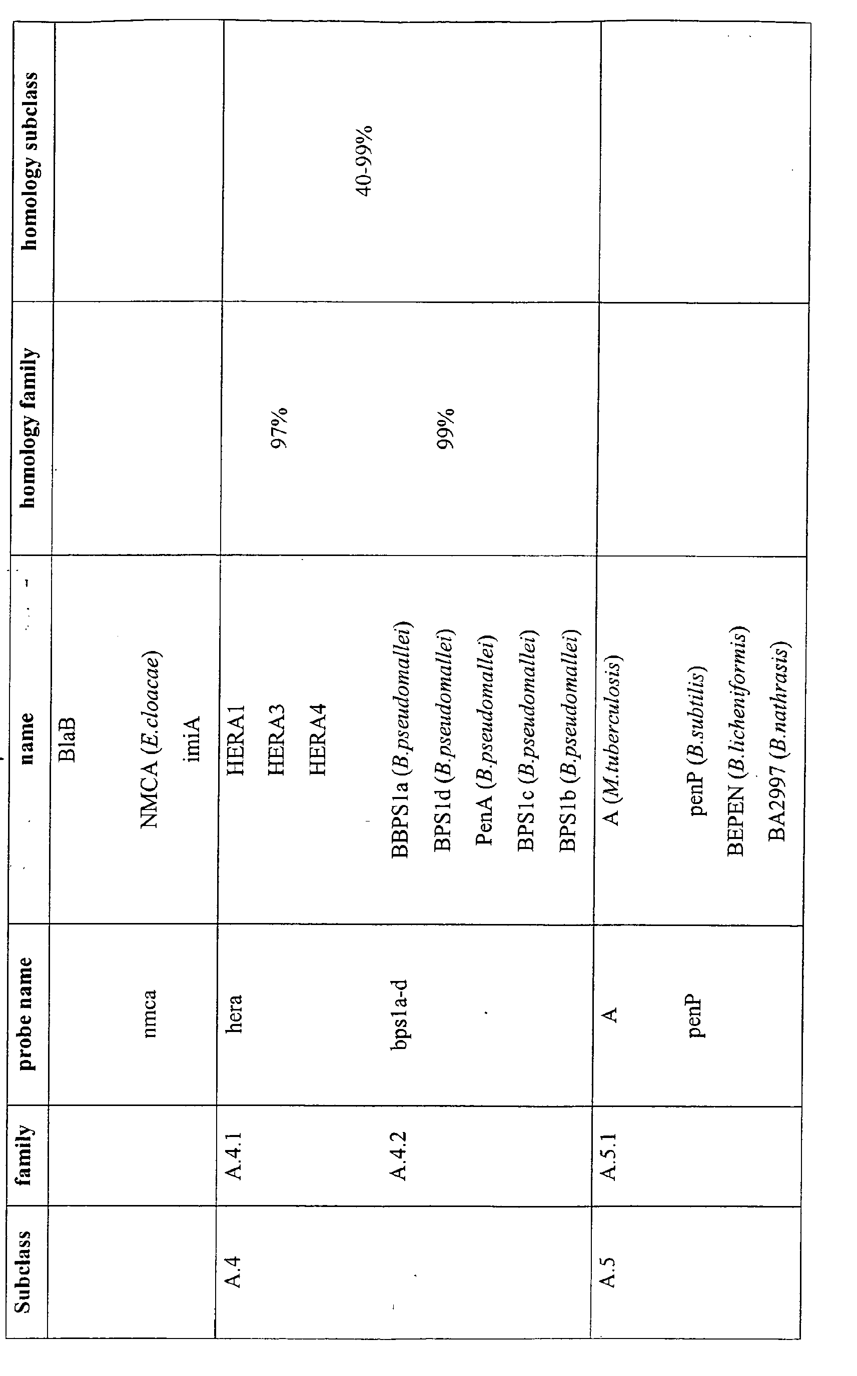

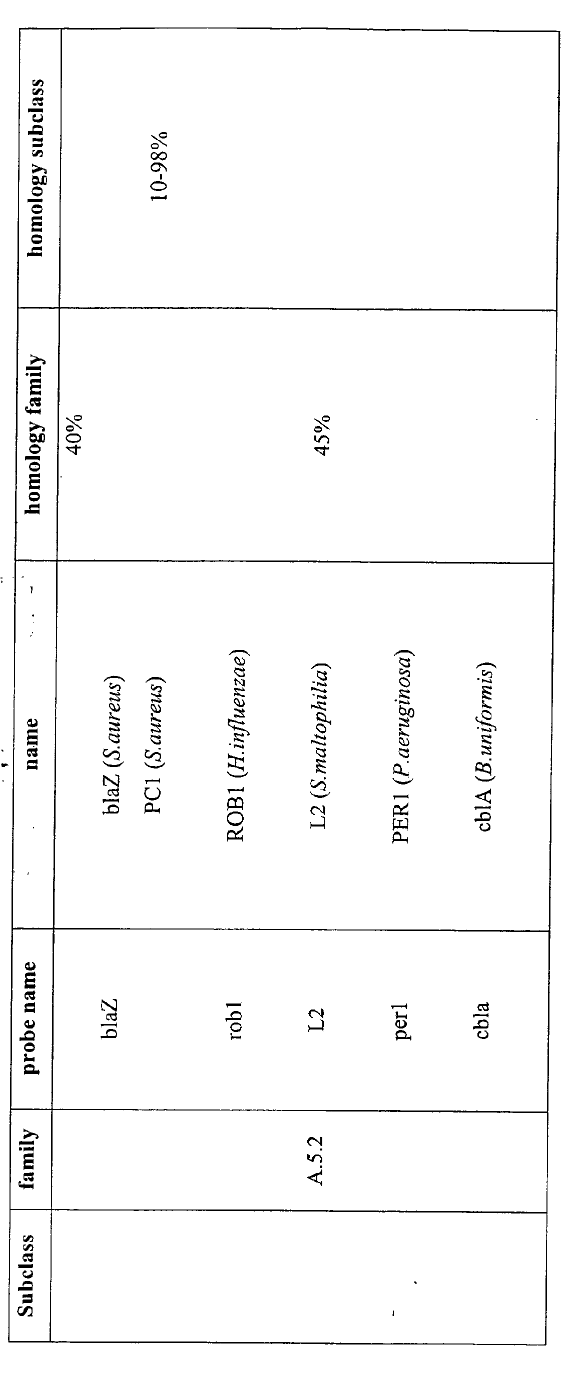

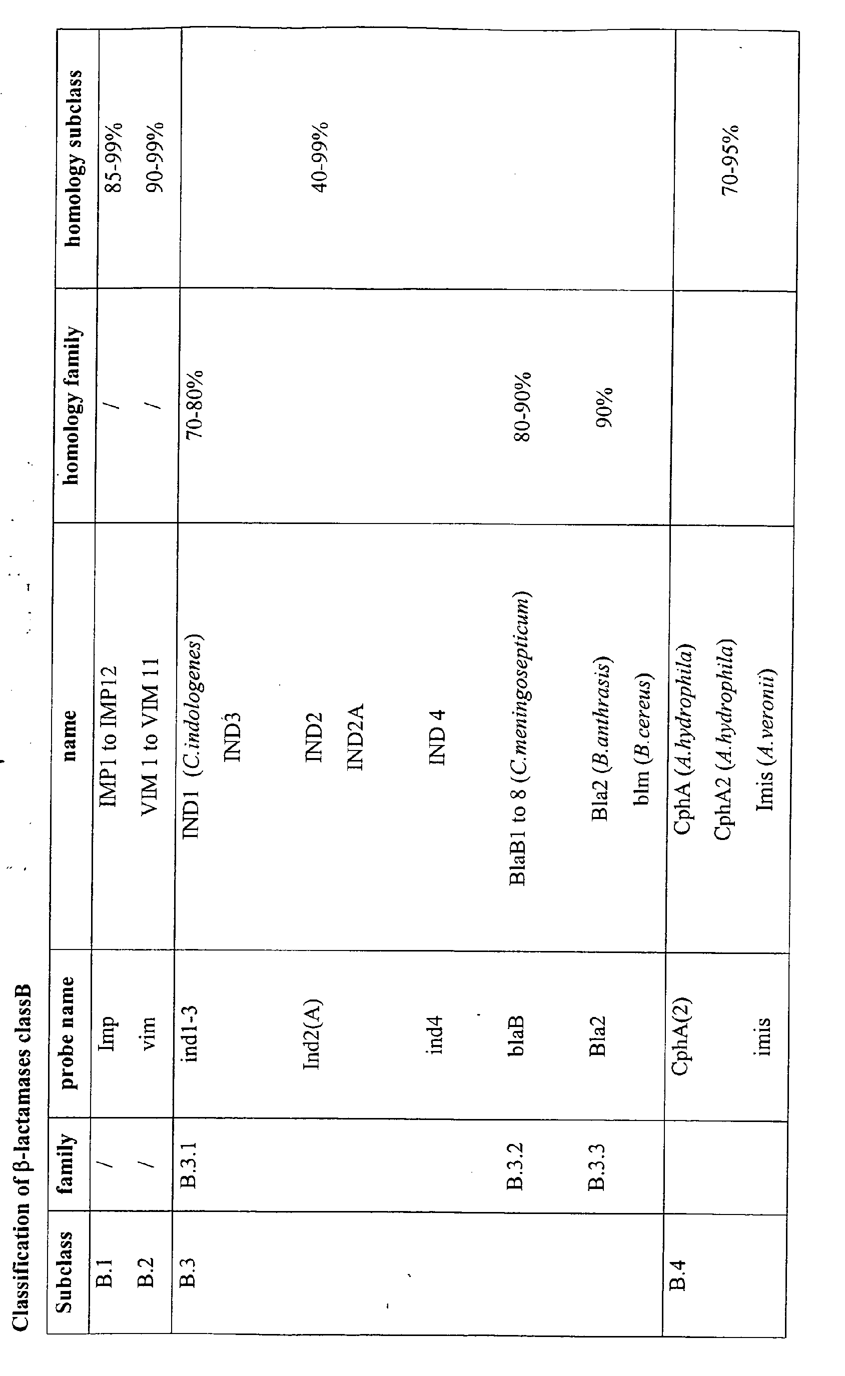

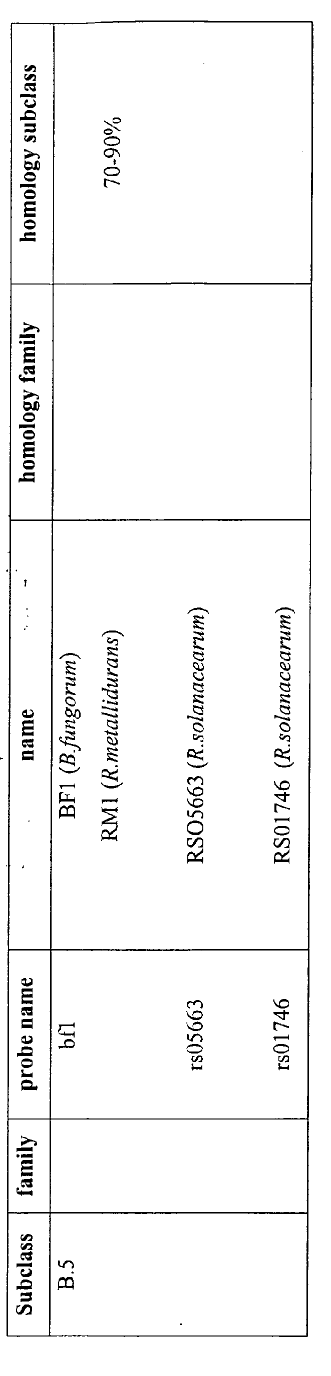

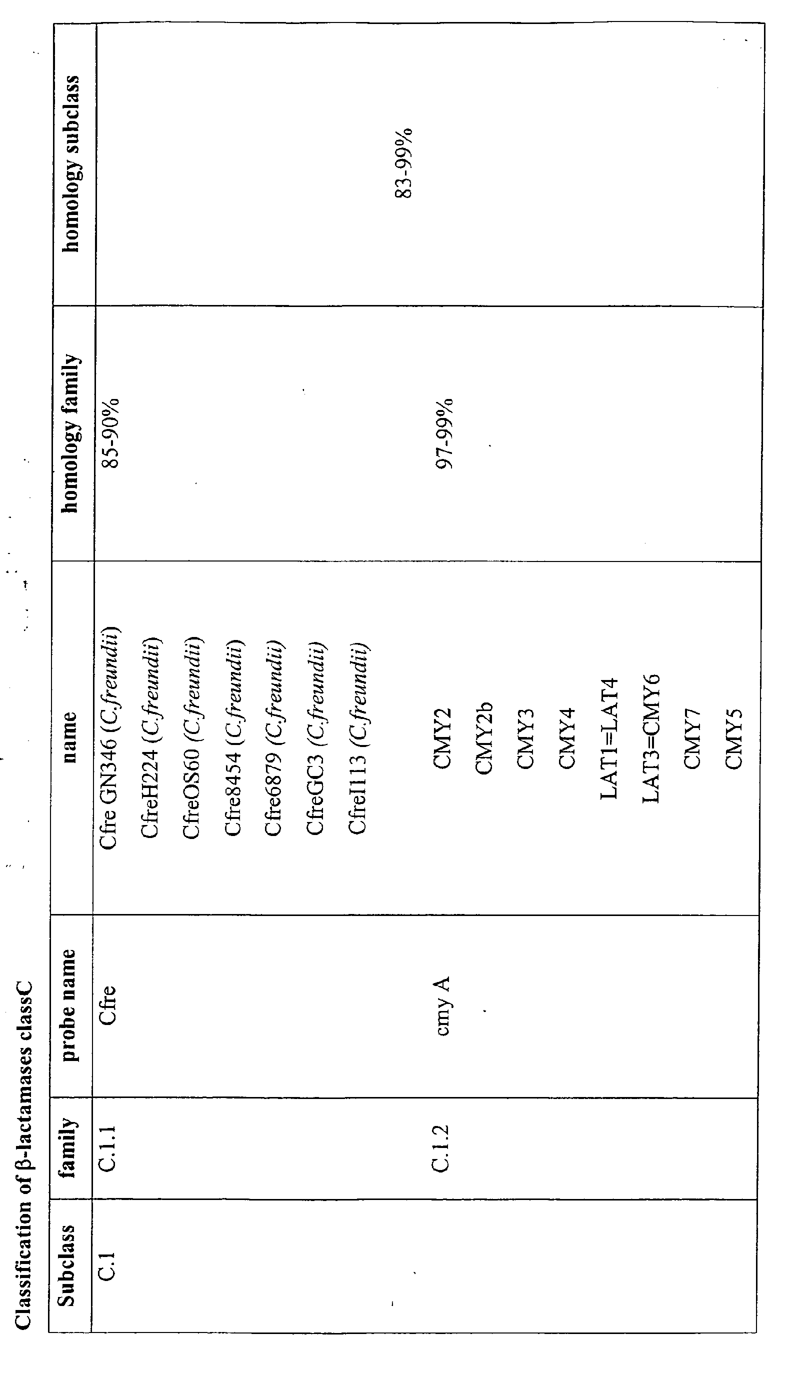

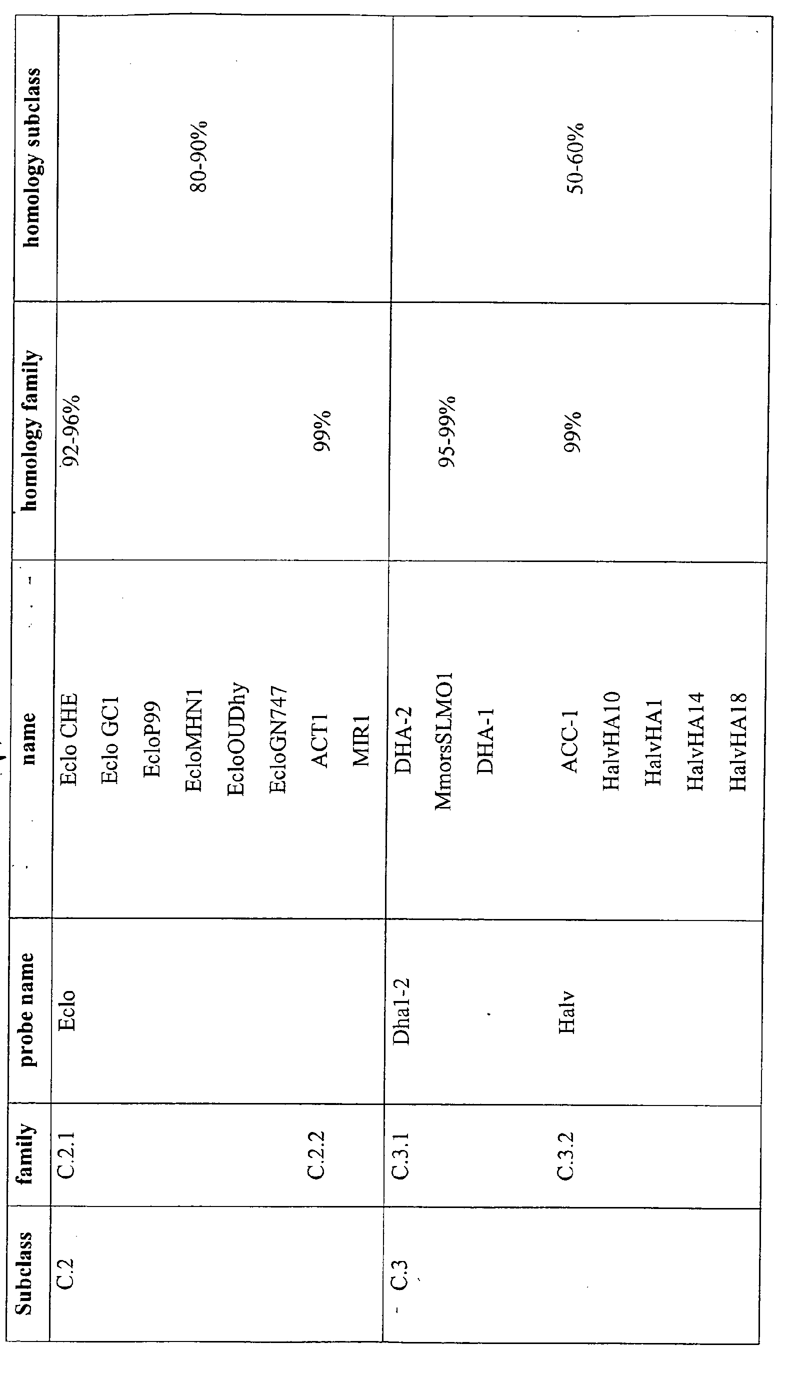

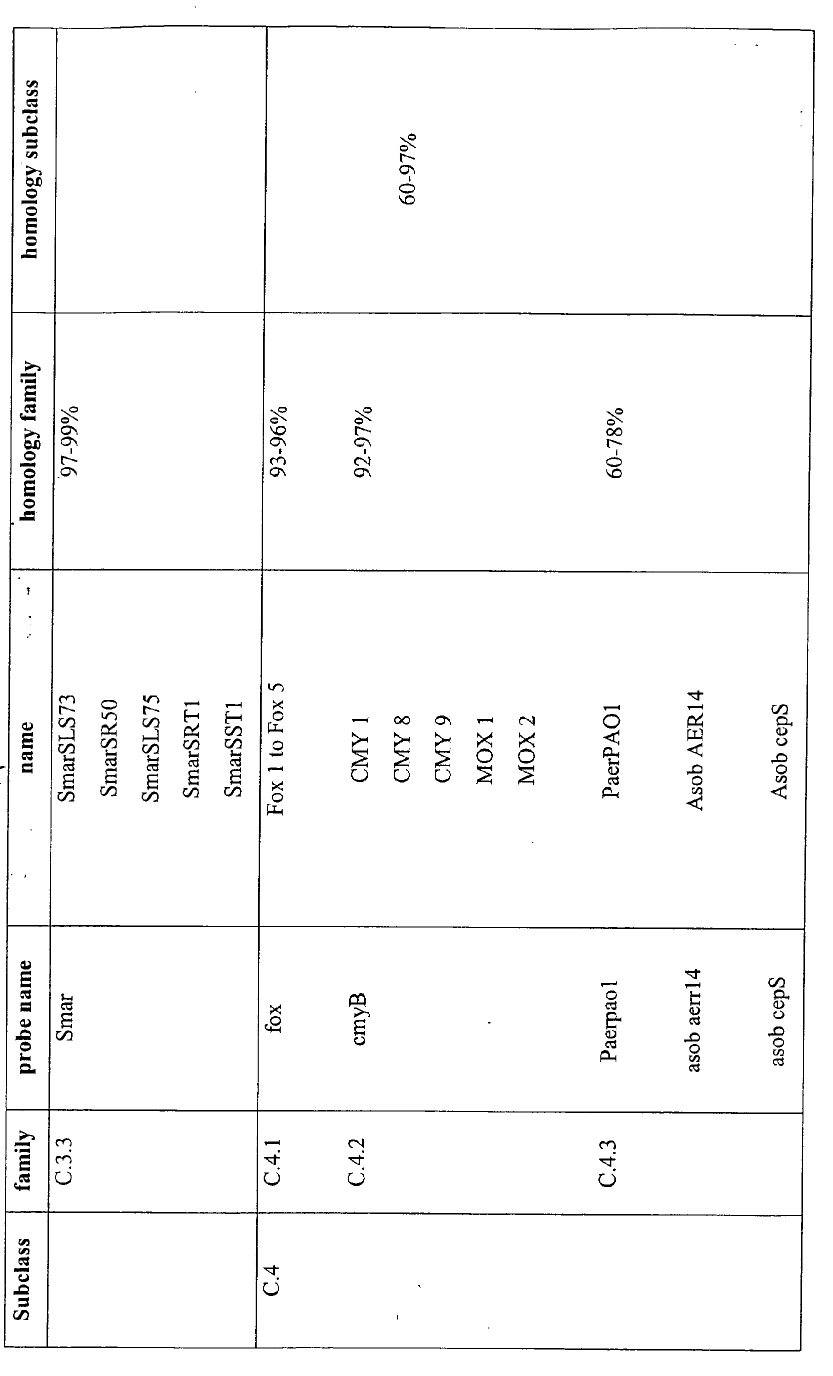

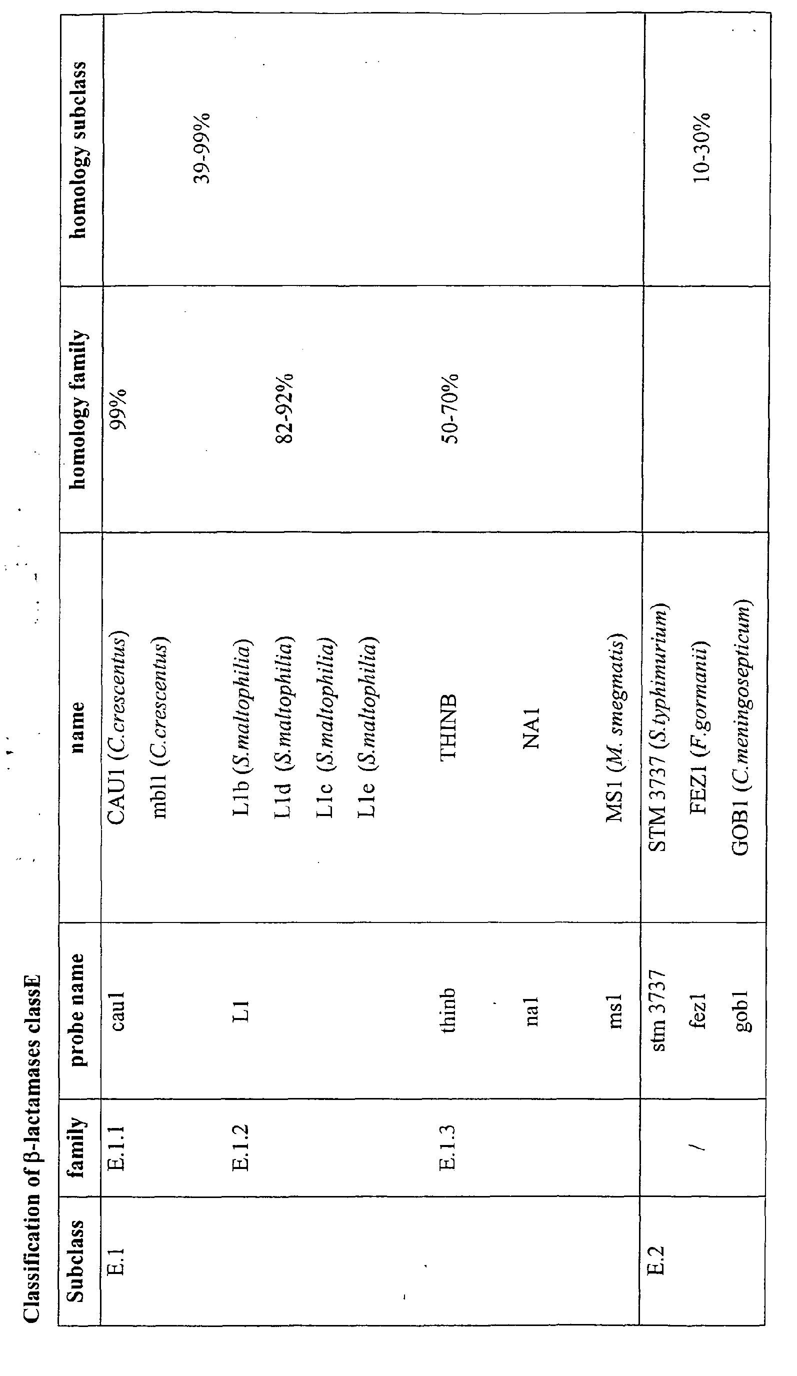

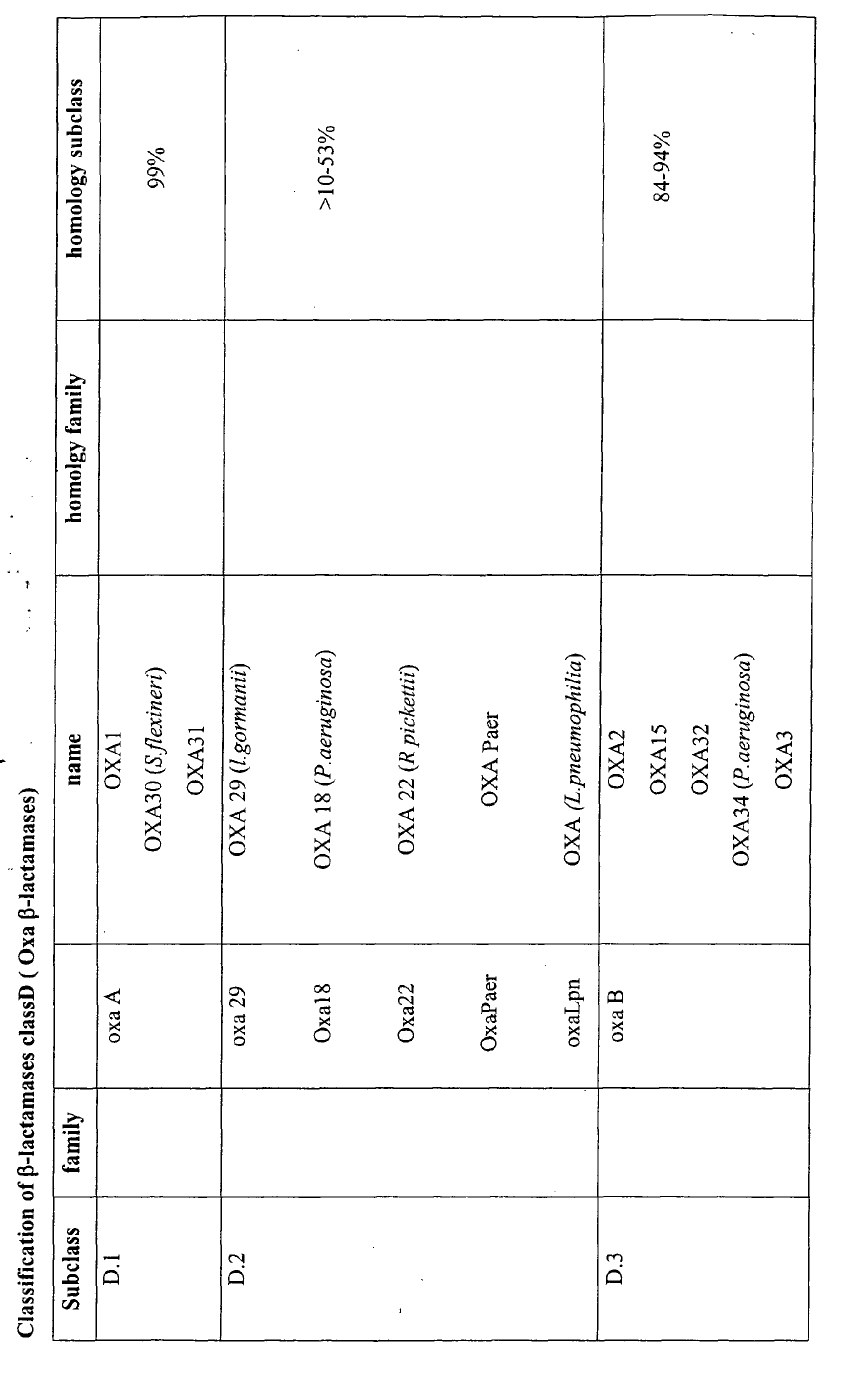

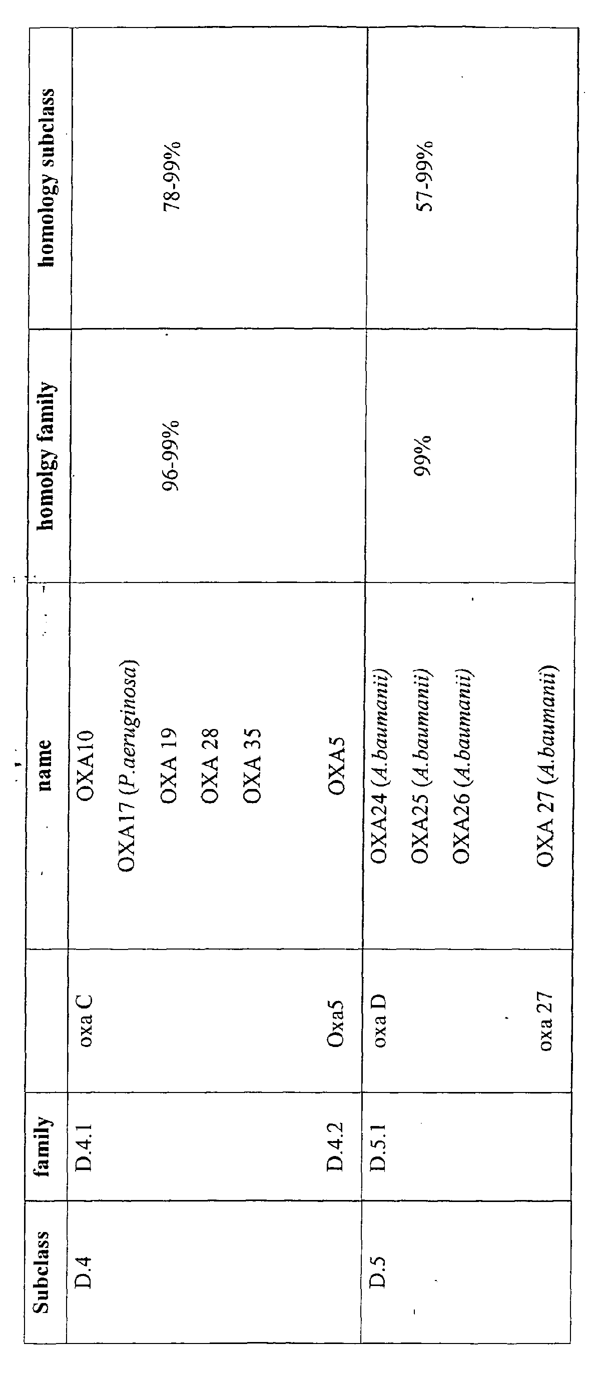

**Table VII Resistance related Gene for different antibiotic: the inactivation enzymes. Inactivating Enzymes**

| Resistance | Protein Name | Gene included (probe name) |
|---|---|---|
| Esterases | | |
| Erythromycin | Ere (A) | ere (A) |
| Erythromycin | Ere (B) | ere (B) |
| Hydrolases | | |
| Streptogramin B | Vgb(A) | vgb(A) |
| Streptogramin B | Vgb(B) | vgb(B) |
| Transferases | | |
| Lincomycin | Lnu (A) a | lnu(A)c |
| Lincomycin | Lnu(B)a | lnu(A)c |
| Streptogramin A | Vat(A) | vat(A) |
| Streptogramin A | Vat (B) | vat (B) |
| Streptogramin A | Vat(C) | vat(C) |
| Streptogramin A | Vat (D) a | vat (D) |
| Streptogramin A | Vat (E) a | vat (E) |
| Phosphorylases | | |
| Macrolides | Mph(A) | mph(A) |
| Macrolides | Mph(B) | mph(B) |
| Macrolides | Mph(C) | mph(C) |

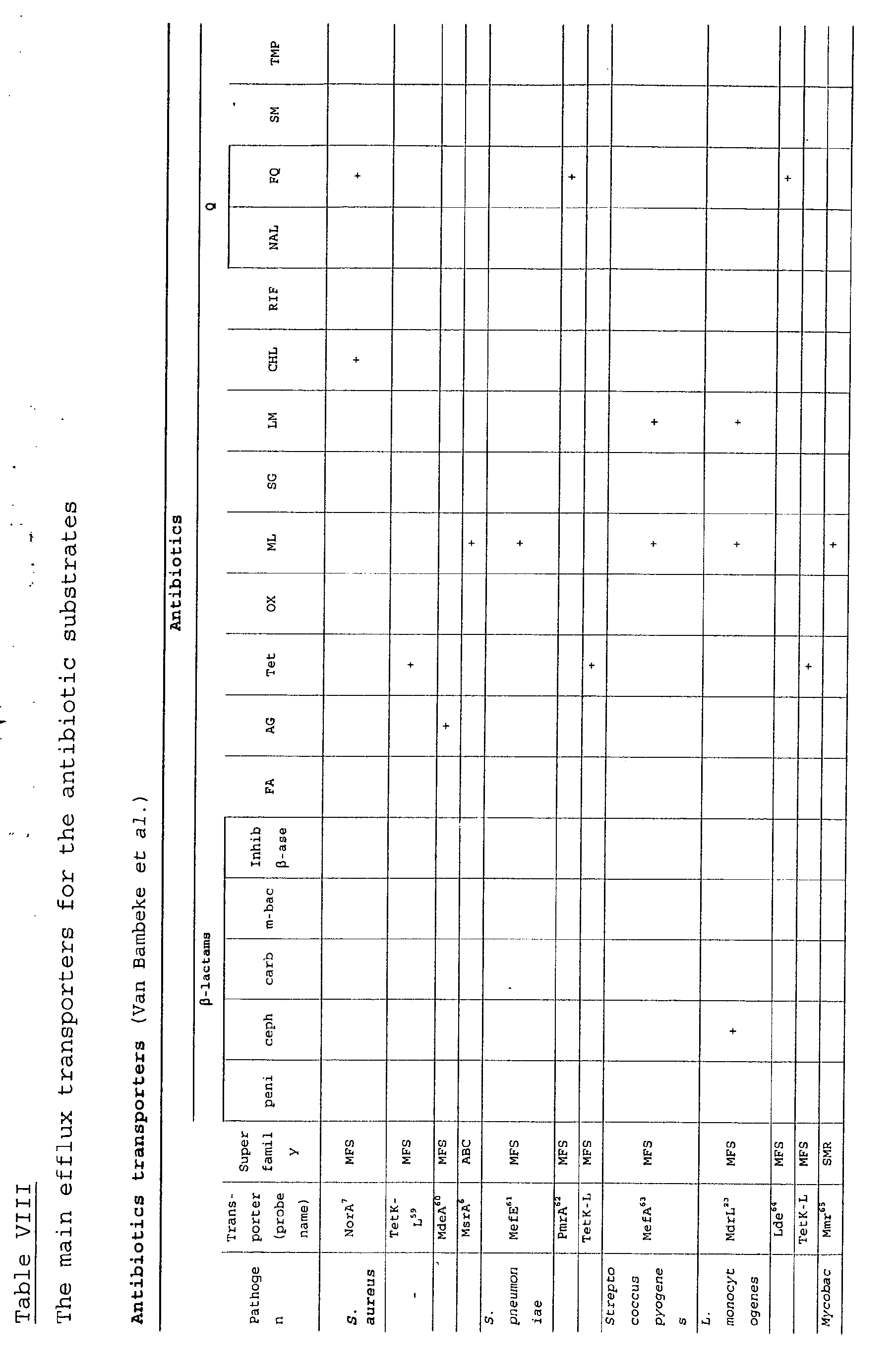

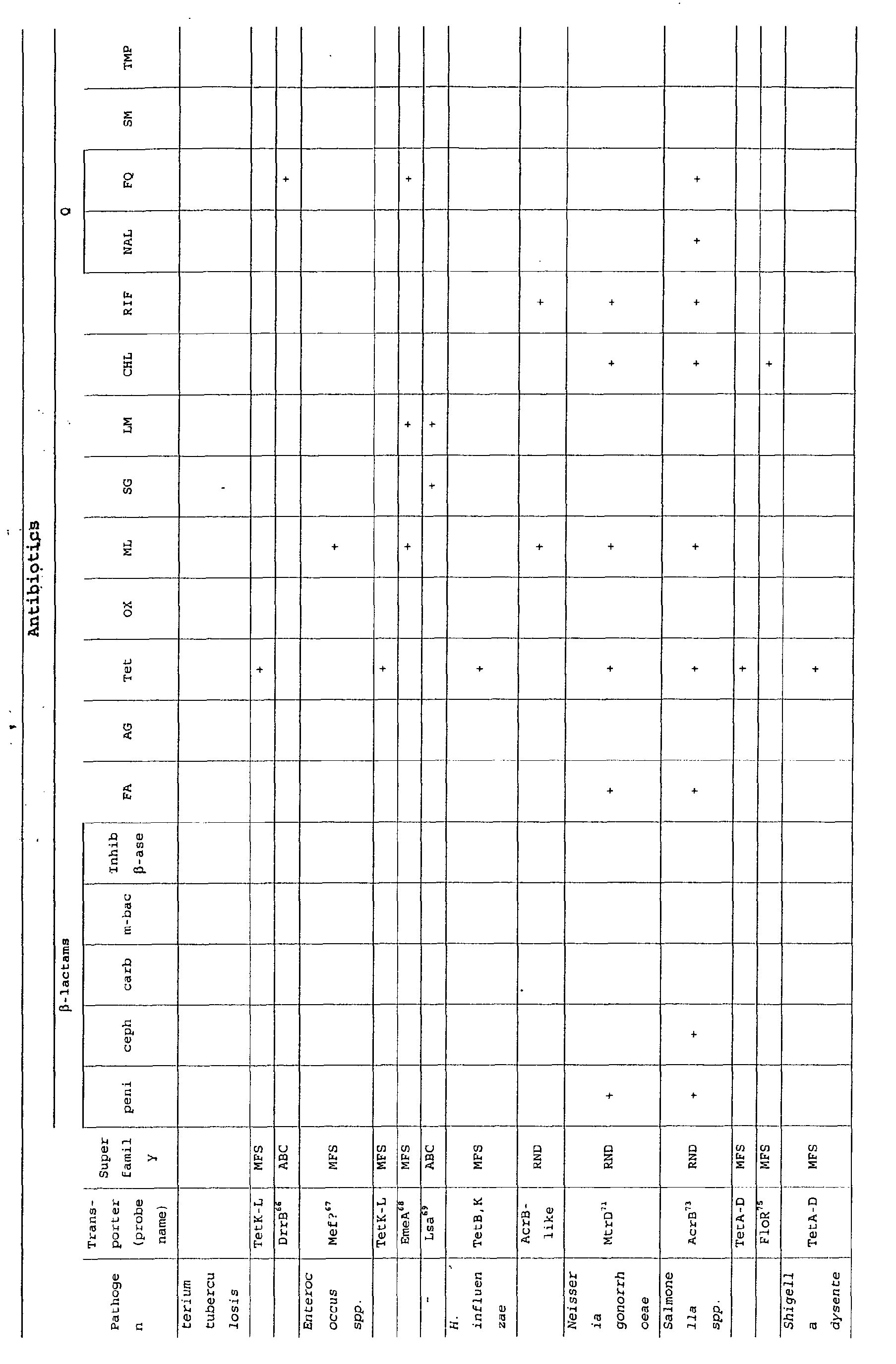

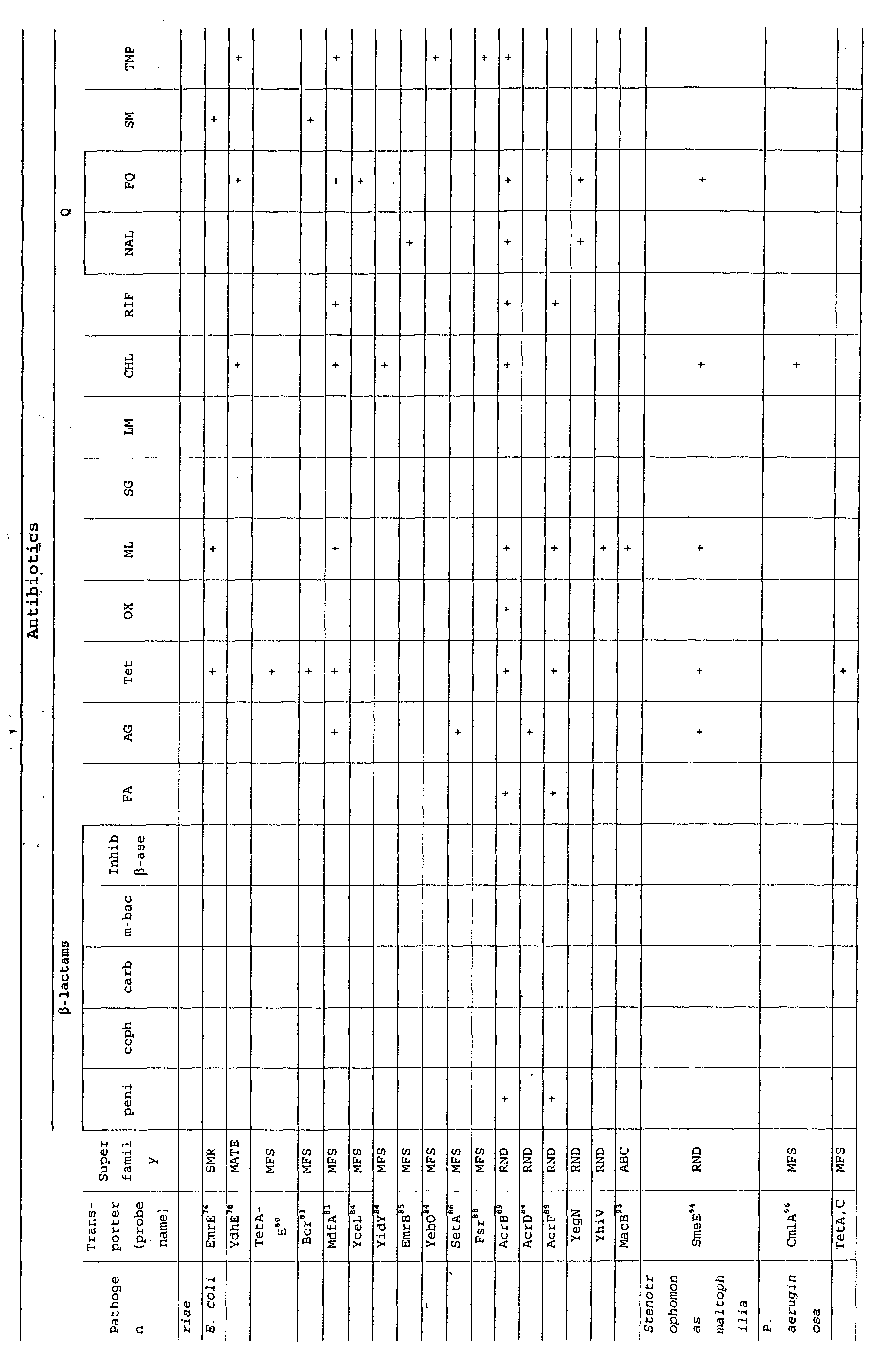

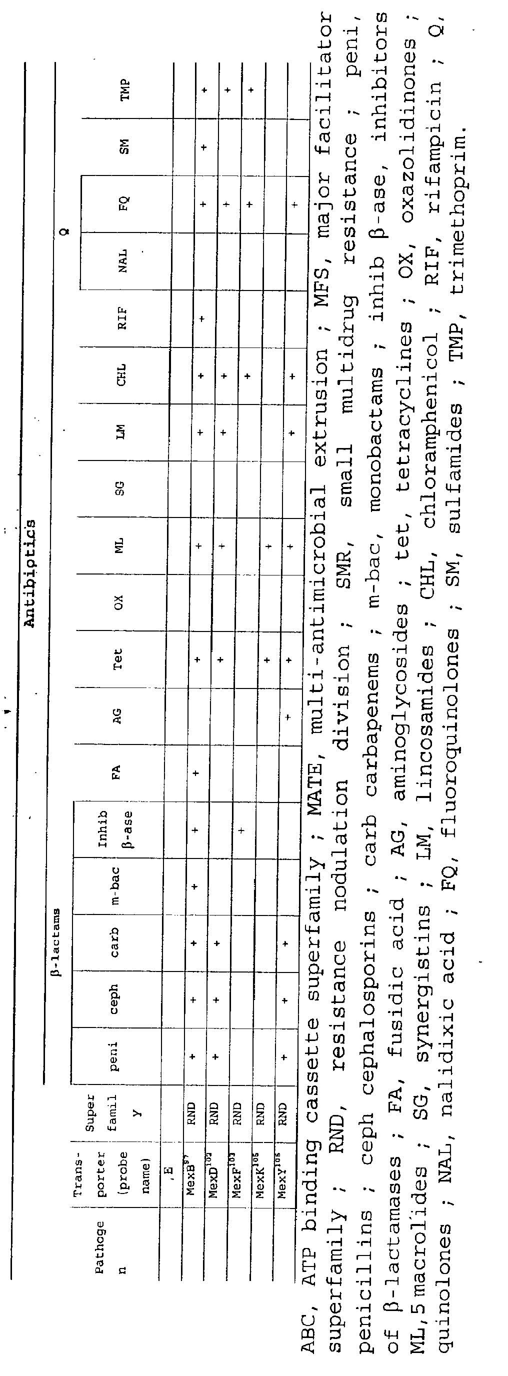

### List of references cited in the patent

Hoopzer, D.C. Emerging mechanisms of fluoroquinolone resistance (2001). *Emerging infectious diseases.* 7, 337-341.

Ruiz, J. Mechanisms of resistance to quinolones : target alterations, decreased accumulation and DNA gyrase protection (2003). *Journal of antimicrobial chemotherapy.* 51, 1109-1117.

Hirai, K., Irikura, T.and Mitsuhashi, S. Differences in susceptibility to quinolones of outer membrane mutants of *Salmonella typhimurium* and *E.coli* (1986). *Antimicrobial agents and chemoterapy.* 535-538.

Van Bambeke, F., Balzi, E., Tulkens, P. Antibiotic efflux pumps. Biochemicals Pharmacology (2000) ; 60 :457-470.

Van Bambeke, F., Glupczynski, Y., Plésiat, P., Pechère, J.C and Tulkens, P.M. Antibiotic efflux in prokaryotic cells : occurrence, impact on resistance and strategies for the future of antimicrobial therapy (2003). Journal of Antimicrobial Chemotherapy 51, 1055-1065

Lage, H. ABC-transporters : implications on drug resistance from microorganisms to human cancers. (2003). International journal of antimicrobial agent 22, 188-199

Webber, M.A, Piddock, J.V. The importance of the efflux pumpsin bacterial antibiotic resistance (2003). Journal of antimicrobial chemotherapy 51, 9-11.

## Claims

1. An identification and/or quantification method of a part of a biological (micro)organism (possibly present in a biological sample) by the detection of expressed gene nucleotide sequence related to resistance to antibiotic(s) and being expressed in the (micro)organism, wherein said expressed gene nucleotide sequence is homologous to at least 4 other expressed gene nucleotide sequences from other biological (micro)organisms, and comprising the steps of:
- extracting the expressed gene nucleotide sequences from the (micro)organism;
- adding enzymatically (a preferably) homopolymeric nucleotide tail to at least one extremity of the said sequences;
- copying and/or amplifying at least part of the said sequences into target homologous nucleotide sequences to be detected by using unique primer(s) which are capable of amplifying and/or copying at least 4 homologous target nucleotide sequences;
- possibly labelling said target nucleotide sequences;
- putting into contact the labelled target nucleotide sequences with single stranded capture nucleotide sequences bound by a single predetermined link to an insoluble solid support, preferably a non-porous solid support;
- discriminating the binding of a target nucleotide sequence specific of a part of an (micro)organism by detecting and/or quantifying and/or recording a signal resulting from a hybridization by complementary base pairing between the target nucleotide sequence and its corresponding capture nucleotide sequence,
wherein the said capture nucleotide sequences are bound to the insoluble solid support at a specific location according to an array having a density of at least 4 different bound single stranded capture nucleotide sequences/cm² of solid support surface and,
wherein the binding between the target nucleotide sequence and its corresponding capture nucleotide sequence forms said signal at the expected location, the detection of a single spot signal allowing to indicate the presence or absence of an expressed nucleotide sequence and predicts a possible antibiotic(s) resistance of the micro(organisms), expressing said nucleotide sequence.

2. The method according to claim 1,
wherein the homologous target nucleotide sequences present between them an homology of sequence (sequence identity) comprised between 2% and 80% and wherein a single stranded capture nucleotide sequence has at least 50 nucleotides which is able to specifically bind to a target nucleotide sequence without any binding to said at least 4 other different homologous nucleotide sequences, having an homology comprised between 2% and 80% with the said target sequence.

3. The method according to claim 2,
wherein the single stranded capture nucleotide sequences have a length of at least 200 nucleotides.

4. The method according to claim 1,
wherein the homologous target nucleotide sequences present between them an homology of sequence higher than 80%,
wherein the single stranded capture nucleotide sequence comprises a specific portion of a nucleotide sequence of about 10 to about 50 nucleotides, which is able to specifically bind to a target nucleotide sequence without binding to said at least 4 other different homologous nucleotide sequences having an homology higher than 80% with the said target nucleotide sequence, and wherein the said specific portion of capture nucleotide sequence is fixed to the solid support surface via a spacer of at least 20 nucleotides.

5. The method according to claim 4,
wherein the spacer is made of at least 40 nucleotides, preferably at least 90 nucleotides.

6. The method according to the claims 4 or 5, wherein the specific portion of the nucleotide sequence has a length comprised between about 15 and about 30 nucleotides, which is able to specifically bind to said target nucleotide.

7. The method according to any of the preceding claims, wherein the steps of copying and/or amplifying at least part of the expressed gene nucleotide sequence into a target homologous nucleotide sequence to be detected is done by using an unique pair of primer(s), preferably by PCR, which are capable of amplifying and/or copying at least 4 homologous target nucleotide sequences.

8. The method according to claim 1 for identifying and/or quantifying an expressed gene nucleotide sequence related to resistance to antibiotic(s) and being expressed in the (micro)organism, among at least 4 different other homologous resistant related genes nucleotide sequences, having between them an homology of sequence (sequence identity), comprised between 2% and 99%,
wherein the array comprises two groups of single-stranded capture nucleotide sequences :
- a first group composed of capture nucleotide sequences having a nucleotide sequence comprised between about 10 and about 50 nucleotides, which is able to specifically bind to a target nucleotide sequence without binding to said at least 4 other different homologous nucleotide sequences having an homology higher than 80% with the said target nucleotide sequence and wherein said first capture nucleotide sequence able to hybridise with its corresponding target nucleotide sequence, is separated from the surface of the solid support by a spacer being a nucleotide sequence having a length of at least 20 nucleotides and,
- a second group composed of capture nucleotide sequences having a sequence of at least 50 nucleotides which is able to specifically bind to a target nucleotide sequence without binding to said at least 4 other different homologous nucleotide sequences having an homology comprised between 2% and 80% with the said target nucleotide sequence.

9. The method according to any of the preceding claims, wherein several expressed gene nucleotide sequences related to resistance to antibiotic(s) being expressed in a (micro)organism and being present in the same sample are simultaneously identified and/or quantified.

10. The method according to claim 9,
wherein at least two expressed gene nucleotide sequences related to resistance to antibiotic(s) are simultaneously identified and/or quantified, said sequences belonging to 2 families, comprising firstly, the sequences specific for one antibiotic and secondly, sequences common for several antibiotics, with at least one sequence being identified and/or quantified in each family.

11. The method according to claim 9 or 10,
wherein at least 10 expressed gene nucleotide sequences related to resistance to antibiotic(s) are simultaneously identified and/or quantified, with at least 6 sequences specific for one antibiotic and at least 4 sequences common for several antibiotics being identified and/or quantified.

12. The method according to claim 10 or 11, wherein the sequences specific for one antibiotic are given in tables I, II, III, V, VI and the sequences common for several antibiotics are given in tables VII and VIII.

13. The method according to any of the preceding claims, wherein the expressed gene nucleotide sequences related to resistance to antibiotic(s) being expressed in a (micro)organism are selected from the group consisting of : methylase enzymes, acetyltransferases, phosphostransferases, adenyltransferases, ligase enzymes, tet proteins, B-lactamase enzymes, inactivating enzymes and efflux transporters.

14. The method according to claim 13, wherein the inactivating enzymes are selected from the group consisting of esterase, hydrolase, transferase and phosphorylase.

15. The method according to any of the preceding claims, wherein the step of copying at least part of the expressed gene nucleotide sequences is obtained with the addition of an unique primer being the complement of homopolymeric nucleotide tail added to one extremity of the expressed gene nucleotide sequences.

16. The method according to claim 15,
wherein the unique primer has a length of at least 10 nucleotides.

17. The method according to claim 15,
wherein the unique primer comprises a T7 RNA promoter nucleotide sequence or its complementary nucleotide strand.

18. The method according to any of the preceding claims, wherein the amplifying step of at least part of expressed gene nucleotide sequences is a in vitro transcription (IVT) or a PCR.

19. The method according to any of the preceding claims, wherein the insoluble solid support is selected from the group consisting of glasses, electronic devices, silicon supports, plastic supports, compact discs, gel layers, metallic supports or a mixture thereof.

20. The method according to any of the preceding claims, which further comprises the step of an identification of a single nucleotide mutation in a target nucleotide sequence by using at least 2 different nucleotide sequences specific for a target nucleotide sequence, said capture nucleotide sequence differing by a single nucleotide.

21. The method according to any of the preceding claims, wherein the target nucleotide sequences are labelled during the copying and/or amplifying step.

22. The method according to any of the preceding claims, wherein the expressed gene nucleotide sequence related to resistance to antibiotic(s) are firstly separated from eucaryotic expressed gene nucleotide sequences.

23. The method according to any of the preceding claims, wherein the amount of capture nucleotide sequence bound to the solid support is higher than 3 fmoles/cm² solid support surface.

24. The method of claim 1, wherein the quantitative measurement is obtained with a data variation coefficient lower than 80%, preferably lower than 25%, more preferably lower than 15%.

25. The method of Claim 24, wherein the quantification measurement is obtained from a average of at least three experimental data.

26. The method according to any of the preceding claims, wherein the array comprises more than 50, but less than 1000 different capture sequences.

27. The method of Claim 26, wherein the array comprises at least captures sequences for the detection and/or the quantification of more than 20, preferably more than 100, or all the expressed gene nucleotide sequences presented in the tables I, II, II, V, VI, VII and VIII.

28. The method according to any of the preceding claims, wherein the biological sample is a clinical sample

29. A diagnostic and/or quantification kit or device comprises an insoluble solid support upon which two groups of single stranded capture nucleotide sequences are bound according to an array,
- wherein said first group being composed of capture nucleotide sequences comprising a specific portion of nucleotide sequence having a length of about 10 to about 50 nucleotides which is able to specifically bind a target nucleotide sequence related to resistance to antibiotic(s) to be identified and/or quantified without binding to at least 4 other homologous and different nucleotide sequences having an homology higher than 80%, with the said nucleotide target sequence; said specific portion of the capture nucleotide sequence being fixed to the solid support surface via a spacer of at least 20 nucleotides;
- wherein said second group being composed of capture nucleotide sequences having a nucleotide sequence of at least 50 nucleotides which is able to specifically bind to a target nucleotide sequence related to resistance to antibiotics to be identified and/or quantified without binding to at least 4 other homologous and different nucleotide sequences having an homology comprised between 2% and 80% with the said target nucleotide sequence,
- wherein the said capture nucleotide sequences are covalently bound to the solid support and,
- wherein said array comprises at least 4 different bound capture nucleotide sequences /cm² fixed at specific locations of the solid support.

30. The diagnostic kit or device according to claim 29, wherein the array further comprises capture nucleotide sequences of the first group for identification of a (micro)organism species and/or genus among at least 4 different (homologous) other (micro)organism species and/or genus.

31. The diagnostic kit or device according to claim 29 or 30, wherein the solid support is selected from the group consisting of glasses, electronic devices, silicon supports, plastic supports, compact discs, gel layers, metallic supports or a mixture thereof.

32. The diagnostic kit or device according to claims 29 to 31, wherein each capture nucleotide sequence is specific to a target nucleotide sequence to be identified and/or quantified and wherein the target nucleotide sequence is an amplified product from an expressed gene nucleotide sequence which is selected from the group consisting of : methylase enzymes, acetyltransferases, phosphostransferases, adenyl-transferases, ligase enzymes, tet proteins, B-lactamase enzymes, inactivating enzymes and efflux transporters.

33. The diagnostic kit or device according to any of the preceding claims 29 to 32, wherein the array further comprises capture nucleotide sequences for the identification of a mutation in a target nucleotide sequence related to resistance to antibiotic(s).

34. A diagnostic and/or quantification kit or device which comprises an insoluble solid support upon which capture nucleotide sequences are bound covalently according to an array comprising at least 4 different bound capture nucleotide sequences / cm² fixed at the solid support surface, said capture nucleotide sequences being fixed at specific locations on the solid support surface, each capture nucleotide sequence being able to specifically bind a target nucleotide sequence being a copy or an amplicon of an expressed gene nucleotide sequence related to resistance to antibiotic(s) to be identified and/or quantified and wherein the capture nucleotide sequences have a length of at least 50 nucleotides.
